# EUROPEAN PATENT APPLICATION

(11) **EP 4 464 781 A1**
(43) Date of publication of application: **20.11.2024**
(21) Application number: 23739951.4
(22) Date of filing: 09.01.2023
(51) Int. Cl.: C12N 15/113, A61K 31/713, A61P 3/06, A61P 35/04

(54) **RNAI AGENT INHIBITING PCSK9 GENE EXPRESSION AND APPLICATION THEREOF**

(30) Priority: 13.01.2022 CN 202210038513; 08.09.2022 CN 202211092636
(71) Applicant: CSPC Zhongqi Pharmaceutical Technology (Shijiazhuang) Co., Ltd., Shijiazhuang, Hebei 050035 (CN)
(72) Inventor: DONG, Yunxia, Shijiazhuang, Hebei 050035 (CN); SHAO, Yu, Shijiazhuang, Hebei 050035 (CN); SU, Xiaoye, Shijiazhuang, Hebei 050035 (CN); WANG, Lingyu, Shijiazhuang, Hebei 050035 (CN)
(74) Representative: Barrow, Nicholas Martin
(86) International application number: PCT/CN2023/071189
(87) International publication number: WO 2023/134609

(57) **Abstract**

An RNAi agent inhibiting PCSK9 gene expression and an application thereof. The present invention relates to a modified double-stranded RNAi agent and an application thereof, and in particular to a double-stranded RNAi agent inhibiting PCSK9 gene expression and a pharmaceutical composition thereof, and an application of the double-stranded RNAi agent or the pharmaceutical composition thereof in the treatment of diseases mediated by PCSK9 expression.

## Description

### Technical Field

The invention belongs to the field of molecular biology and relates to a modified double-stranded RNAi agent and the use thereof. Specifically, it relates to a double-stranded RNAi agent that inhibits PCSK9 gene expression and a pharmaceutical composition thereof, as well as use of the double-stranded RNAi agent or the pharmaceutical composition thereof for the treatment of a disease mediated by PCSK9 expression.

### Background

RNA interference (RNAi) widely exists in natural species. After Andrew Fire and Craig Mello et al. first discovered the RNAi phenomenon in *Caenorhabditis elegans* (*C. elegans*) in 1998, and Tuschl and Phil Sharp et al. confirmed its existence in mammals in 2001, a series of progress has been made in research on the mechanism, gene function and clinical application of RNAi. RNAi plays a key role in various body protection mechanisms such as the defense against viral infection and the prevention of transposon jumping (Hutvágner et al., 2001; Elbashire et al., 2001; Zamore 2001). Products developed based on the RNAi mechanism are very promising candidate drugs. Small interfering RNA (siRNA) can exert RNA interference and is the main tool to achieve RNAi.

A proprotein convertase, subtilisin/kexin-9 (also known as PCSK9) is a serine protease that indirectly regulates plasma LDL cholesterol level by controlling the expression of hepatic and extrahepatic LDL receptors (LDLR) on plasma membrane. Reduced PCSK9 protein expression increases LDLR receptor expression, thereby reducing plasma LDL cholesterol and resulting hypercholesterolemia and/or atherosclerosis and complications induced thereby. At the same time, studies have found that mice with PCSK9 knockout have reduced blood cholesterol level and show enhanced sensitivity to statins in reducing blood cholesterol. The above studies show that PCSK9 inhibitors may be beneficial in reducing LDL-C concentration in the blood and in treating PCSK9-mediated diseases, and are therefore expected to become potential therapeutic targets for controlling hypercholesterolemia and its complications.

At present, the clinical treatment of cholesterolemia and its complications mainly relies on statin small molecule drugs. Studies have shown that patients who are intolerant to statin drugs can cause myopathy and other adverse reactions, such as myalgia and rhabdomyolysis. Although evolocumab (trade name: Rebain) is currently on the market in China, it is expensive, and PCSK9 monoclonal antibody is metabolized by the reticuloendothelial system and requires injection every 2-4 weeks. Studies have shown that small interfering RNA (siRNA) can specifically silence the PCSK9 gene, and thereby inhibits its protein expression and reduces low-density lipoprotein (LDL-c). At the same time, the drug Inclisiran approved in the Europe and the United States is bringing hope to patients with hypercholesterolemia due to its durable efficacy (a subcutaneous injection every six months). Therefore, the development of an efficient inhibitor that silences PCSK9 will provide an effective means for long-term treatment of hypercholesterolemia, making it have better efficacy, specificity, stability, targeting or tolerability, etc.

### Summary of the invention

The object of the present invention is to provide a double-stranded RNAi agent that inhibits PCSK9 gene expression and a pharmaceutical composition thereof, and a method and use of the above-mentioned double-stranded RNAi agent and the pharmaceutical composition thereof for inhibiting or reducing PCSK9 gene expression or treating a PCSK9 expression-mediated disease or symptom.

One embodiment of the present invention provides a double-stranded RNAi agent that can inhibit the expression of PCSK9 in a cell, wherein the double-stranded RNAi agent comprises a sense strand and an antisense strand, wherein the sense strand is complementary to the antisense strand, and the antisense strand comprises a sequence complementary to a portion of the mRNA encoding PCSK9, wherein each strand is 14 to 30 nucleotides in length, and the sense strand nucleotide sequence in the double-stranded RNAi agent is selected from 14 to 30 nucleotides in SEQ ID NO:1 or SEQ ID NO:2.

For example, each strand (sense or antisense) can be 14-30 nucleotides in length, 17-30 nucleotides in length, 25-30 nucleotides in length, 27-30 nucleotides in length, 17-23 nucleotides in length, 17-21 nucleotides in length, 17-19 nucleotides in length, 19-25 nucleotides in length, 19-23 nucleotides in length, 19-21 nucleotides in length, 21-25 nucleotides in length, or 21-23 nucleotides in length.

The duplex region of the double-stranded RNAi agent can be, for example, 14-30 nucleotide pairs in length, 17-30 nucleotide pairs in length, 27-30 nucleotide pairs in length, 17-23 nucleotide pairs in length, 17-21 nucleotide pairs in length, 17-19 nucleotide pairs in length, 19-25 nucleotide pairs in length, 19-23 nucleotide pairs in length, 19-21 nucleotide pairs in length, 21-25 nucleotide pairs in length, or 21-23 nucleotide pairs in length.

In another example, the duplex region has a length selected from the group consisting of 15, 16, 17, 18, 19, 20, 21, 22, 23, 24, 25, 26, and 27 nucleotide pairs.

In one embodiment of the present invention, the double-stranded RNAi agent can inhibit PCSK9 gene expression in a human, a monkey, a rat or a mouse.

The RNAi agent of the present invention include a RNAi agent having a nucleotide overhang at one end (i.e., an agent having an overhang and a blunt end) or having a nucleotide overhang at both ends.

In one embodiment, the double-stranded RNAi agent may comprise one or more overhang regions and/or capping groups at the 3' end, the 5' end, or both ends of one or both strands. The overhang can be 1-6 nucleotides in length, such as 2-6 nucleotides in length, 1-5 nucleotides in length, 2-5 nucleotides in length, 1-4 nucleotides in length, 2-4 nucleotides in length, 1-3 nucleotides in length, 2-3 nucleotides in length, or 1-2 nucleotides in length, and the overhang is arbitrarily selected from U, A, G, C, T.

In one embodiment, the sense strand of the double-stranded RNAi agent has 21 nucleotides and the antisense strand has 23 nucleotides.

In another example, one or more nucleotides in the sense and antisense strands of the double-stranded RNAi agent have one or more modifications selected from the group consisting of: 2'-methoxyethyl, 2'-O-alkyl, 2'-O-allyl, 2'-C-allyl, 2'-fluoro, 2'-deoxy, 2'-hydroxy, locked nucleic acid modification, ring-opening or non-locked nucleic acid modification, DNA modification, fluorescent probe modification.

In one embodiment of the invention, both the sense strand and the antisense strand of the double-stranded RNAi agent comprise 2'-O-methyl and/or 2'-fluoro modifications.

In another example of the present invention, the double-stranded RNAi agent further comprises at least one phosphorothioate or methylphosphonate internucleotide bond, preferably at least one phosphorothioate bond.

In another example of the present invention, in the double-stranded RNAi agent, the phosphorothioate or methylphosphonate internucleotide bond is at the 5' and 3' ends of one strand, preferably, the inter-nucleotide bond is at the 5' and 3' ends of the sense strand and the antisense strand; more preferably, the inter-nucleotide bond is between 3 nucleotides at the 5' and 3' ends of the sense strand and antisense strand.

In another embodiment of the invention, the double-stranded RNAi agent comprises: (1) the antisense strand has an overhang of 5'(s)mN(s)mN3' structure at the 3' end; (2) the antisense strand is modified with fluoro at least at positions 2, 6, 14, and 16 from the 5' end, and is modified with methoxy as far as possible at other positions; (3) the antisense strand is modified with at least two thio modifications starting from the 3' end and the 5' end; (4) the sense strand is modified with fluoro at position 7 and positions 9-11continuously from the 5' end, and other positions are modified with methoxy as far as possible; (5) the sense strand has at least two thio modifications starting from the 5' end, and GalNAc is used for covalent coupling at the 3' end.

In another embodiment of the invention, the double-stranded RNAi agent comprises: (1) a sense strand having 21 nucleotides, consisting of alternating 2'-fluoro modified regions and 2'-O-methyl modified regions. The length of each modified region is 1 to 3 nucleotides; the first modified region from the 5' end and that from the 3' end are modified in the same way; (2) an antisense strand having 23 nucleotides, consisting of alternating 2'-O-methyl modified regions and 2'-fluoro modified regions. The length of each modified region is 1 to 3 nucleotides, and the continuous nucleotide region from positions 1 to 3 from the 5' end and that from the 3' end are both linked by a phosphorothioate backbone.

In one embodiment of the invention, the double-stranded RNAi agent is conjugated to at least one ligand selected from the group consisting of cholesterol, biotin, vitamins, galactose derivatives or analogs, lactose derivatives or analogs, N-acetylgalactosamine derivatives or analogs, N-acetylglucosamine (GalNAc) derivatives or analogs.

In some embodiments, the ligand is linked to the 3' end, 5' end and/or in the middle of the sequence of the double-stranded RNAi agent.

In some embodiments, the above-mentioned double-stranded RNAi agent is modified with 1-5, 2-4 or 3 N-acetylgalactosamine derivatives or analogs (X) at the 3' end, 5' end and/or in the middle of the sequence. Specifically, the structure of a single N-acetylgalactosamine derivative is shown in Formula I: 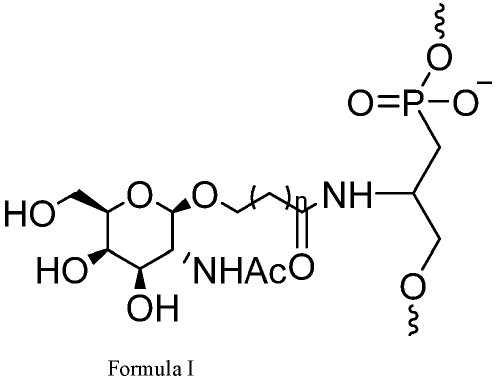 wherein n is an integer from 1 to 15.

In one embodiment of the present invention, XX means two adjacent Xs linked through a phosphodiester bond or a phosphorothioate diester bond, XXX means three adjacent Xs linked through phosphodiester bonds or phosphorothioate diester bonds, and XXXX mean four adjacent Xs linked through phosphodiester bonds or phosphorothioate diester bonds. In the XX structure, the values of n in the two X structures are equal; in the XXX structure, the values of n in the three X structures are equal; in the XXXX structure, the values of n in the four X structures are equal; specifically, n is 3 or 1.

Preferably, the ligand is linked to the 3' end of the sense strand.

In one example of the invention, in the double-stranded RNAi agent, the ligand is one or more GalNAc derivatives linked to a monovalent or trivalent branched linker.

In one example of the invention, the double-stranded RNAi agent comprises:
(1) an antisense strand consisting of nucleotide sequence UfsGmsUfCmCfUmCfUmCfUfGfUmUfGmCfCmUfUmUfUmUfand and a sense strand consisting of nucleotide sequence AmsAfsAmAfAmGfGmCfAmAfCmAmGmAfGmAfGmGfAmCfAmsGmsAm;
(2) an antisense strand consisting of nucleotide sequence GfsUmsCfCmUfCmUfCmUfGfUfUmGfCmCfUmUfUmUfUmAfand and a sense strand consisting of nucleotide sequence UmsAfsAmAfAmAfGmGfCmAfAmCmAmGfAmGfAmGfGmAfCmsAmsGm;
(3) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(4) an antisense strand consisting of nucleotide sequence GfsAmsCfCmUfGmUfUmUfUfGfCmUfUmUfUmGfUmAfAmCfand and a sense strand consisting of nucleotide sequence GmsUfsUmAfCmAfAmAfAmGfCmAmAmAfAmCfAmGfGmUfCmsUmsAm;
(5) an antisense strand consisting of nucleotide sequence AfsCmsCfUmGfUmUfUmUfGfCfUmUfUmUfGmUfAmAfCmUfand and a sense strand consisting of nucleotide sequence AmsGfsUmUfAmCfAmAfAmAfGmCmAmAfAmAfCmAfGmGfUmsCmsUm;
(6) an antisense strand consisting of nucleotide sequence CfsUmsGfUmUfUmUfGmCfUfUfUmUfGmUfAmAfCmUfUmGfand and a sense strand consisting of nucleotide sequence CmsAfsAmGfUmUfAmCfAmAfAmAmGmCfAmAfAmAfCmAfGmsGmsUm;
(7) an antisense strand consisting of nucleotide sequence UfsUmsUfGmUfAmAfCmUfUfGfAmAfGmAfUmAfUmUfUmAfand and a sense strand consisting of nucleotide sequence UmsAfsAmAfUmAfUmCfUmUfCmAmAmGfUmUfAmCfAmAfAmsAmsGm;
(8) an antisense strand consisting of nucleotide sequence UfsUmsGfUmAfAmCFUmUfGfAfAmGfAmUfAmUfUmUfAmUfand and a sense strand consisting of nucleotide sequence AmsUfsAmAfAmUfAmUfCmUfUmCmAmAfGmUfUmAfCmAfAmsAmsAm;
(9) an antisense strand consisting of nucleotide sequence UfsGmsUfAmAfCmUfUmGfAfAfGmAfUmAfUmUfUmAfUmUfand and a sense strand consisting of nucleotide sequence AmsAfsUmAfAmAfUmAfUmCfUmUmCmAfAmGfUmUfAmCfAmsAmsAm;
(10) an antisense strand consisting of nucleotide sequence GfsUmsAfAmCfUmUfGmAfAfGfAmUfAmUfUmUfAmUfUmCfand and a sense strand consisting of nucleotide sequence GmsAfsAmUfAmAfAmUfAmUfCmUmUmCfAmAfGmUfUmAfCmsAmsAm;
(11) an antisense strand consisting of nucleotide sequence AfsUmsAfUmUfUmAfUmUfCfUfGmGfGmUfUmUfUmGfUmAfand and a sense strand consisting of nucleotide sequence UmsAfsCmAfAmAfAmCfCmCfAmGmAmAfUmAfAmAfUmAfUmsCmsUm;
(12) an antisense strand consisting of nucleotide sequence UfsUmsUfAmUfUmCfUmGfGfGfUmUfUmUfGmUfAmGfCmAfand and a sense strand consisting of nucleotide sequence UmsGfsCmUfAmCfAmAfAmAfCmCmCmAfGmAfAmUfAmAfAmsUmsAm;
(13) an antisense strand consisting of nucleotide sequence AfsUmsUfCmUfGmGfGmUfUfUfUmGfUmAfGmCfAmUfUmUfand and a sense strand consisting of nucleotide sequence AmsAfsAmUfGmCfUmAfCmAfAmAmAmCfCmCfAmGfAmAfUmsAmsAm;
(14) an antisense strand consisting of nucleotide sequence CfsUmsGfGmGfUmUfUmUfGfUfAmGfCmAfUmUfUmUfUmAfand and a sense strand consisting of nucleotide sequence Um s Afs Am AfAm AfUm GfCmUfAm Cm Am AfAm AfCm CfCm AfGm s Am s Am;
(15) an antisense strand consisting of nucleotide sequence UfsGmsGfGmUfUmUfUmGfUfAfGmCfAmUfUmUfUmUfAmUfand and a sense strand consisting of nucleotide sequence AmsUfsAmAfAmAfAmUfGmCfUmAmCmAfAmAfAmCfCmCfAmsGmsAm;
(16) an antisense strand consisting of nucleotide sequence GfsGmsGfCmUfGmAfGmCfUfUfUmAfAmAfAmUfGmGfUmUfand and a sense strand consisting of nucleotide sequence Am s Afs Cm CfAmUfUmUfUm AfAm Am Gm CfUm CfAm GfCm CfCm s Cm s Am;
(17) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(18) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(19) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(20) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(21) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAmsGm;
(22) an antisense strand consisting of nucleotide sequence
   AfsGmsAfCmCfUmGfUmUfUfUfGmCfUmUfUmUfGmUfAmAfGmCmAmGmCmCmdGd AGmGmCmUmsGmsCmand and a sense strand consisting of nucleotide sequence UmsUfsAmCfAmAfAmAfGmCfAmAmAmAfCmAfGmGfUmCfUmsAmsGm;
(23) an antisense strand consisting of nucleotide sequence AfsGmsAfCmCfUmGfUmUfUfUfGmCfUmUfUmUfGmUfAmAfand and a sense strand consisting of nucleotide sequence UmsUfsAmCfAmAfAmAfGmCfAmAmAmAfCmAfGmGfUmCfUmsAfsGm;
(24) an antisense strand consisting of nucleotide sequence AfsGmsAfCmCfUmGfUmUfUfUfGmCfUmUmUmUmGmUmAmAmand and a sense strand consisting of nucleotide sequence UmsUfsAmCfAmAmAmAfGmCfAmAmAmAfCmAfGmGmUmCfUmsAmsGm;
(25) an antisense strand consisting of nucleotide sequence AmsGmsAmCmCmUmGfUmUfUmUmGmCmUmUmUmUmGmUmAmAmand and a sense strand consisting of nucleotide sequence UmsUfsAmCfAmAmAmAfGmCfAmAmAmAfCmAfGmGmUmCfUmsAmsGm;
(26) an antisense strand consisting of nucleotide sequence AmsGmsAmCmCmUmGfUmUfUmUmGmCmUmUmUmUmGmUmAmAmand and a sense strand consisting of nucleotide sequence UmsUfsAmCfAfAfAmAfGmCfAmAfAmAfCmAfGmGfUmCmUmsAmsGm;
(27) an antisense strand consisting of nucleotide sequence AmsGmsAmCmCmUmGfUmUfUm(dT)GmCmUmUmUmUmGmUmAmAmand and a sense strand consisting of nucleotide sequence UmsUfsAmCfAfAfAmAfGmCfAmAfAmAfCmAfGmGfUmCmUmsAmsGm;
(28) an antisense strand consisting of nucleotide sequence CfsUmsGfUmUfUmUfGmCfUfUfUmUfGmUfAmAfCmUfUmGfand and a sense strand consisting of nucleotide sequence CmsAfsAmGfUmUfAmCfAmAfAmAmGmCfAmAfAmAfCmAfGmsGfsUm;
(29) an antisense strand consisting of nucleotide sequence CfsUmsGfUmUfUmUfGmCfUfUfUmUfGmUmAmAmCmUmUmGmand and a sense strand consisting of nucleotide sequence CmsAfsAmGfUmUmAmCfAmAfAmAmGmCfAmAfAmAmCmAfGmsGmsUm;
(30) an antisense strand consisting of nucleotide sequence CmsUmsGmUmUmUmUfGmCfUmUmUmUmGmUmAmAmCmUmUmGmand and a sense strand consisting of nucleotide sequence CmsAfsAmGfUmUmAmCfAmAfAmAmGmCfAmAfAmAmCmAfGmsGmsUm;
(31) an antisense strand consisting of nucleotide sequence CmsUmsGmUmUmUmUfGmCfUmUmUmUmGmUmAmAmCmUmUmGmand and a sense strand consisting of nucleotide sequence CmsAfsAmGfUfUfAmCfAmAfAmAfGmCfAmAfAmAfCmAmGmsGmsUm;
(32) an antisense strand consisting of nucleotide sequence CmsUmsGmUmUmUmUfGmCfUm(dT)UmUmGmUmAmAmCmUmUmGmand and a sense strand consisting of nucleotide sequence CmsAfsAmGfUfUfAmCfAmAfAmAfGmCfAmAfAmAfCmAmGmsGmsUm;
(33) an antisense strand consisting of nucleotide sequence CmsUmsGmUmUmUmUfGmCfUfUfUmUmGmUmAmAmCmUmUmGmand and a sense strand consisting of nucleotide sequence CmsAfsAmGmUmUfAmCfAfAmAmAmGmCfAmAfAmAmCmAmGmsGmsUm;
(34) an antisense strand consisting of nucleotide sequence CmsUmsGmUmUmUmUfGmCfUfUfUmUmGmUmAmAmCmUmUmGmand and a sense strand consisting of nucleotide sequence CmsAfsAmGmUmUfAmCmAmAmAmAmGmCfAmAfAmAmCmAmGmsGmsUm; or
(35) an antisense strand consisting of nucleotide sequence CmsUmsGmUmUmUmUfGmCfUfUfUmUmGmUmAmAmCmUmUmGmand and a sense strand consisting of nucleotide sequence CmsAfsAmGmUmUmAmCmAmAmAmAmGmCfAmAfAmAmCmAmGmsGmsUm;
   wherein Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone.

In some examples of the invention, the double-stranded RNAi agent comprises:
(1) an antisense strand consisting of a nucleotide sequence having at least 90%, preferably 95%, 96%, 97%, 98%, or 99% identity with nucleotide sequence UfsGmsUfCmCfUmCfUmCfUfGfUmUfGmCfCmUfUmUfUmUf and a sense strand consisting of a nucleotide sequence having at least 90%, preferably 95%, 96%, 97%, 98%, or 99% identity with nucleotide sequence AmsAfsAmAfAmGfGmCfAmAfCmAmGmAfGmAfGmGfAmCfAmsGmsAm;
(2) an antisense strand consisting of a nucleotide sequence having at least 90%, preferably 95%, 96%, 97%, 98%, or 99% identity with nucleotide sequence GfsUmsCfCmUfCmUfCmUfGfUfUmGfCmCfUmUfUmUfUmAf and a sense strand consisting of a nucleotide sequence having at least 90%, preferably 95%, 96%, 97%, 98%, or 99% identity with nucleotide sequence UmsAfsAmAfAmAfGmGfCmAfAmCmAmGfAmGfAmGfGmAfCmsAmsGm;
(3) an antisense strand consisting of a nucleotide sequence having at least 90%, preferably 95%, 96%, 97%, 98%, or 99% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, or 99% identity with nucleotide sequence UmsUfsAmCfAmAfAmAfGmCfAmAmAmAfCmAfGmGfUmCfUmsAmsGm;
(4) an antisense strand consisting of a nucleotide sequence having at least 90%, preferably 95%, 96%, 97%, 98%, or 99% identity with nucleotide sequence GfsAmsCfCmUfGmUfUmUfUfGfCmUfUmUfUmGfUmAfAmCf and a sense strand consisting of a nucleotide sequence having at least 90%, preferably 95%, 96%, 97%, 98%, or 99% identity with nucleotide sequence GmsUfsUmAfCmAfAmAfAmGfCmAmAmAfAmCfAmGfGmUfCmsUmsAm;
(5) an antisense strand consisting of a nucleotide sequence having at least 90%, preferably 95%, 96%, 97%, 98%, or 99% identity with nucleotide sequence AfsCmsCfUmGfUmUfUmUfGfCfUmUfUmUfGmUfAmAfCmUf and a sense strand consisting of a nucleotide sequence having at least 90%, preferably 95%, 96%, 97%, 98%, or 99% identity with nucleotide sequence AmsGfsUmUfAmCfAmAfAmAfGmCmAmAfAmAfCmAfGmGfUmsCmsUm;
(6) an antisense strand consisting of a nucleotide sequence having at least 90%, preferably 95%, 96%, 97%, 98%, or 99% identity with nucleotide sequence CfsUmsGfUmUfUmUfGmCfUfUfUmUfGmUfAmAfCmUfUmGf and a sense strand consisting of a nucleotide sequence having at least 90%, preferably 95%, 96%, 97%, 98%, or 99% identity with nucleotide sequence CmsAfsAmGfUmUfAmCfAmAfAmAmGmCfAmAfAmAfCmAfGmsGmsUm;
(7) an antisense strand consisting of a nucleotide sequence having at least 90%, preferably 95%, 96%, 97%, 98%, or 99% identity with nucleotide sequence UfsUmsUfGmUfAmAfCmUfUfGfAmAfGmAfUmAfUmUfUmAf and a sense strand consisting of a nucleotide sequence having at least 90%, preferably 95%, 96%, 97%, 98%, or 99% identity with nucleotide sequence UmsAfsAmAfUmAfUmCfUmUfCmAmAmGfUmUfAmCfAmAfAmsAmsGm;
(8) an antisense strand consisting of a nucleotide sequence having at least 90%, preferably 95%, 96%, 97%, 98%, or 99% identity with nucleotide sequence UfsUmsGfUmAfAmCfUmUfGfAfAmGfAmUfAmUfUmUfAmUf and a sense strand consisting of a nucleotide sequence having at least 90%, preferably 95%, 96%, 97%, 98%, or 99% identity with nucleotide sequence AmsUfsAmAfAmUfAmUfCmUfUmCmAmAfGmUfUmAfCmAfAmsAmsAm;
(9) an antisense strand consisting of a nucleotide sequence having at least 90%, preferably 95%, 96%, 97%, 98%, or 99% identity with nucleotide sequence UfsGmsUfAmAfCmUfUmGfAfAfGmAfUmAfUmUfUmAfUmUf and a sense strand consisting of a nucleotide sequence having at least 90%, preferably 95%, 96%, 97%, 98%, or 99% identity with nucleotide sequence AmsAfsUmAfAmAfUmAfUmCfUmUmCmAfAmGfUmUfAmCfAmsAmsAm;
(10) an antisense strand consisting of a nucleotide sequence having at least 90%, preferably 95%, 96%, 97%, 98%, or 99% identity with nucleotide sequence GfsUmsAfAmCfUmUfGmAfAfGfAmUfAmUfUmUfAmUfUmCf and a sense strand consisting of a nucleotide sequence having at least 90%, preferably 95%, 96%, 97%, 98%, or 99% identity with nucleotide sequence GmsAfsAmUfAmAfAmUfAmUfCmUmUmCfAmAfGmUfUmAfCmsAmsAm;
(11) an antisense strand consisting of a nucleotide sequence having at least 90%, preferably 95%, 96%, 97%, 98%, or 99% identity with nucleotide sequence AfsUmsAfUmUfUmAfUmUfCfUfGmGfGmUfUmUfUmGfUmAf and a sense strand consisting of a nucleotide sequence having at least 90%, preferably 95%, 96%, 97%, 98%, or 99% identity with nucleotide sequence UmsAfsCmAfAmAfAmCfCmCfAmGmAmAfUmAfAmAfUmAfUmsCmsUm;
(12) an antisense strand consisting of a nucleotide sequence having at least 90%, preferably 95%, 96%, 97%, 98%, or 99% identity with nucleotide sequence UfsUmsUfAmUfUmCfUmGfGfGfUmUfUmUfGmUfAmGfCmAf and a sense strand consisting of a nucleotide sequence having at least 90%, preferably 95%, 96%, 97%, 98%, or 99% identity with nucleotide sequence UmsGfsCmUfAmCfAmAfAmAfCmCmCmAfGmAfAmUfAmAfAmsUmsAm;
(13) an antisense strand consisting of a nucleotide sequence having at least 90%, preferably 95%, 96%, 97%, 98%, or 99% identity with nucleotide sequence AfsUmsUfCmUfGmGfGmUfUfUfUmGfUmAfGmCfAmUfUmUf and a sense strand consisting of a nucleotide sequence having at least 90%, preferably 95%, 96%, 97%, 98%, or 99% identity with nucleotide sequence AmsAfsAmUfGmCfUmAfCmAfAmAmAmCfCmCfAmGfAmAfUmsAmsAm;
(14) an antisense strand consisting of a nucleotide sequence having at least 90%, preferably 95%, 96%, 97%, 98%, or 99% identity with nucleotide sequence CfsUmsGfGmGfUmUfUmUfGfUfAmGfCmAfUmUfUmUfUmAf and a sense strand consisting of a nucleotide sequence having at least 90%, preferably 95%, 96%, 97%, 98%, or 99% identity with nucleotide sequence Um s Afs Am AfAm AfUm GfCmUfAm Cm Am AfAm AfCm CfCm AfGm s Am s Am;
(15) an antisense strand consisting of a nucleotide sequence having at least 90%, preferably 95%, 96%, 97%, 98%, or 99% identity with nucleotide sequence UfsGmsGfGmUfUmUfUmGfUfAfGmCfAmUfUmUfUmUfAmUf and a sense strand consisting of a nucleotide sequence having at least 90%, preferably 95%, 96%, 97%, 98%, or 99% identity with nucleotide sequence AmsUfsAmAfAmAfAmUfGmCfUmAmCmAfAmAfAmCfCmCfAmsGmsAm;
(16) an antisense strand consisting of a nucleotide sequence having at least 90%, preferably 95%, 96%, 97%, 98%, or 99% identity with nucleotide sequence GfsGmsGfCmUfGmAfGmCfUfUfUmAfAmAfAmUfGmGfUmUf and a sense strand consisting of a nucleotide sequence having at least 90%, preferably 95%, 96%, 97%, 98%, or 99% identity with nucleotide sequence Am s Afs Cm CfAmUfUmUfUm AfAm Am Gm CfUm CfAm GfCm CfCm s Cm s Am;
(17) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(18) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(19) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(20) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(21) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence Um sUfs Am CfAm AfAm AfGm CfAm Am Am AfCm AfGm GfUm CfUm s Am s Gm;
(22) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAfGmCmAmGmCmCmdGdAG mGmCmUmsGmsCm and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(23) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and an antisense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAfsGm;
(24) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUmUmUmGmUmAmAm and an antisense strand consisting of a nucleotide sequence having at least 90%, preferably 95%, 96%, 97%, 98%, 99% or 100% identity with nucleotide sequence UmsUfsAmCfAmAmAmAfGmCfAmAmAmAfCmAfGmGmUmCfUmsAmsGm;
(25) an antisense strand consisting of a nucleotide sequence having at least 90%, preferably 95%, 96%, 97%, 98%, 99% or 100% identity with nucleotide sequence AmsGmsAmCmCmUmGfUmUfUmUmGmCmUmUmUmUmGmUmAmAm and an antisense strand consisting of a nucleotide sequence having at least 90%, preferably 95%, 96%, 97%, 98%, 99% or 100% identity with nucleotide sequence UmsUfsAmCfAmAmAmAfGmCfAmAmAmAfCmAfGmGmUmCfUmsAmsGm;
(26) an antisense strand consisting of a nucleotide sequence having at least 90%, preferably 95%, 96%, 97%, 98%, 99% or 100% identity with nucleotide sequence AmsGmsAmCmCmUmGfUmUfUmUmGmCmUmUmUmUmGmUmAmAm and an antisense strand consisting of a nucleotide sequence having at least 90%, preferably 95%, 96%, 97%, 98%, 99% or 100% identity with nucleotide sequence UmsUfsAmCfAfAfAmAfGmCfAmAfAmAfCmAfGmGfUmCmUmsAmsGm;
(27) an antisense strand consisting of a nucleotide sequence having at least 90%, preferably 95%, 96%, 97%, 98%, 99% or 100% identity with nucleotide sequence AmsGmsAmCmCmUmGfUmUfUm(dT)GmCmUmUmUmUmGmUmAmAm and an antisense strand consisting of a nucleotide sequence having at least 90%, preferably 95%, 96%, 97%, 98%, 99% or 100% identity with nucleotide sequence UmsUfsAmCfAfAfAmAfGmCfAmAfAmAfCmAfGmGfUmCmUmsAmsGm;
(28) an antisense strand consisting of a nucleotide sequence having at least 90%, preferably 95%, 96%, 97%, 98%, 99% or 100% identity with nucleotide sequence CfsUmsGfUmUfUmUfGmCfUfUfUmUfGmUfAmAfCmUfUmGf and an antisense strand consisting of a nucleotide sequence having at least 90%, preferably 95%, 96%, 97%, 98%, 99% or 100% identity with nucleotide sequence CmsAfsAmGfUmUfAmCfAmAfAmAmGmCfAmAfAmAfCmAfGmsGfsUm;
(29) an antisense strand consisting of a nucleotide sequence having at least 90%, preferably 95%, 96%, 97%, 98%, 99% or 100% identity with nucleotide sequence CfsUmsGfUmUfUmUfGmCfCTfCTfCTmUfGmUmAmAmCmUmUmGm and an antisense strand consisting of a nucleotide sequence having at least 90%, preferably 95%, 96%, 97%, 98%, 99% or 100% identity with nucleotide sequence CmsAfsAmGfUmUmAmCfAmAfAmAmGmCfAmAfAmAmCmAfGmsGmsUm;
(30) an antisense strand consisting of a nucleotide sequence having at least 90%, preferably 95%, 96%, 97%, 98%, 99% or 100% identity with nucleotide sequence CmsUmsGmUmUmUmUfGmCfCTmUmUmUmGmUmAmAmCmUmUmGm and an antisense strand consisting of a nucleotide sequence having at least 90%, preferably 95%, 96%, 97%, 98%, 99% or 100% identity with nucleotide sequence CmsAfsAmGfUmUmAmCfAmAfAmAmGmCfAmAfAmAmCmAfGmsGmsUm;
(31) an antisense strand consisting of a nucleotide sequence having at least 90%, preferably 95%, 96%, 97%, 98%, 99% or 100% identity with nucleotide sequence CmsUmsGmUmUmUmUfGmCfCTmUmUmUmGmUmAmAmCmUmUmGm and an antisense strand consisting of a nucleotide sequence having at least 90%, preferably 95%, 96%, 97%, 98%, 99% or 100% identity with nucleotide sequence Cm s Afs Am GfUfUfAm CfAm AfAm AfGm CfAm AfAm AfCm Am Gm s Gm sUm;
(32) an antisense strand consisting of a nucleotide sequence having at least 90%, preferably 95%, 96%, 97%, 98%, 99% or 100% identity with nucleotide sequence CmsUmsGmUmUmUmUfGmCfUm(dT)UmUmGmUmAmAmCmUmUmGm and an antisense strand consisting of a nucleotide sequence having at least 90%, preferably 95%, 96%, 97%, 98%, 99% or 100% identity with nucleotide sequence Cm s Afs Am GfUfUfAm CfAm AfAm AfGm CfAm AfAm AfCm Am Gm s Gm sUm;
(33) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and an antisense strand consisting of a nucleotide sequence having at least 90%, preferably 95%, 96%, 97%, 98%, 99% or 100% identity with nucleotide sequence CmsAfsAmGmUmUfAmCfAfAmAmAmGmCfAmAfAmAmCmAmGmsGmsUm;
(34) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and an antisense strand consisting of a nucleotide sequence having at least 90%, preferably 95%, 96%, 97%, 98%, 99% or 100% identity with nucleotide sequence CmsAfsAmGmUmUfAmCmAmAmAmAmGmCfAmAfAmAmCmAmGmsGmsUm; (35) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and an antisense strand consisting of a nucleotide sequence having at least 90%, preferably 95%, 96%, 97%, 98%, 99% or 100% identity with nucleotide sequence CmsAfsAmGmUmUmAmCmAmAmAmAmGmCfAmAfAmAmCmAmGmsGmsUm;
wherein Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone.

In another example of the present invention, in the double-stranded RNAi agent, the ligand structure is shown in Formula II:

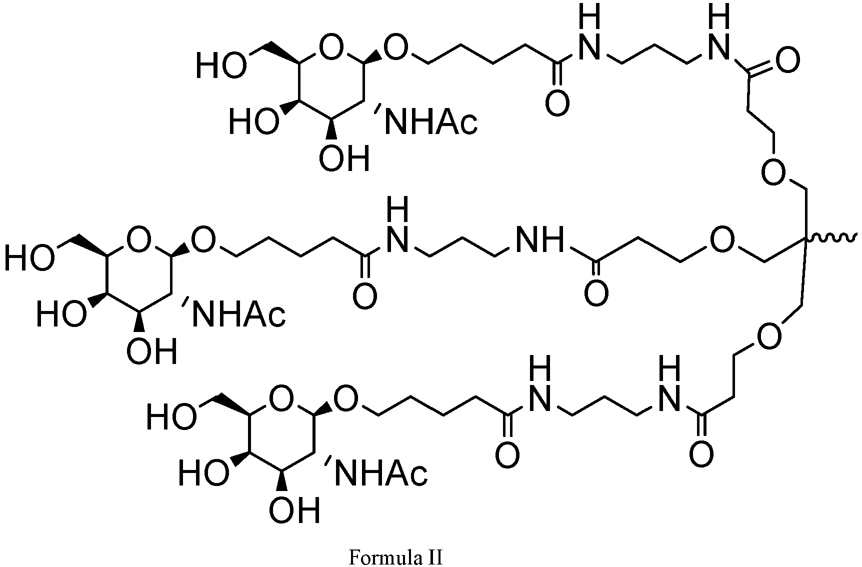

In one example of the present invention, the double-stranded RNAi agent has the structure of formula III, wherein X is O or S:

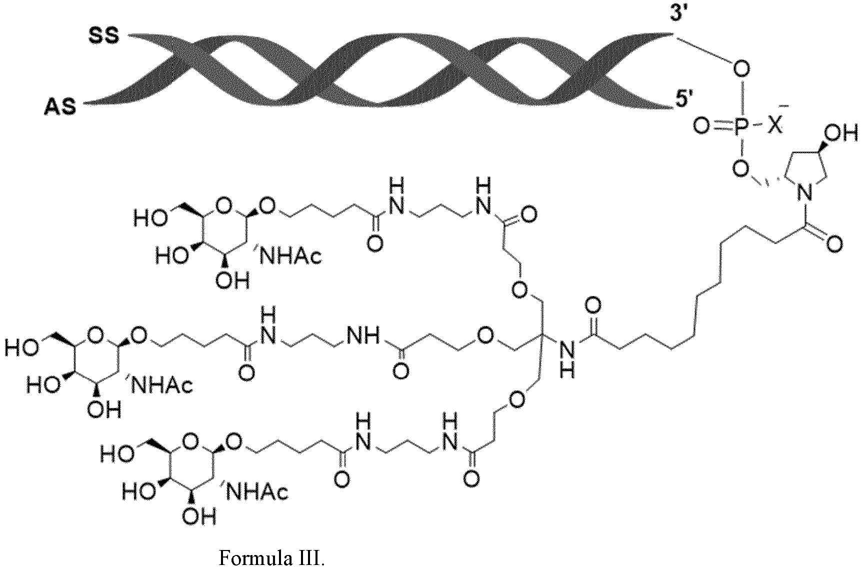

The present invention provides the following examples of double-stranded RNAi agents: E1128, E1129, E1164, E1164-GN1, E1164-GN2, E1164-GN3, E1164-GN4, E1164-GN5, E1164-GN6, E1165, E1166, E1168, E1178, E1179, E1180, E1181, E1192, E1195, E1198, E1201, E1202, E0044, 1164E01, 1164E02, 1164E03, 1164E04, 1164E05, 1164E06, 1168E01, 1168E02, 1168E03, 1168 E04, 1168E05, 1168E06, 1168E07, 1168E08, 1168E09.

The present invention also includes a DNA molecule capable of producing the above-mentioned double-stranded RNAi agent, a vector capable of expressing the double-stranded RNAi agent, and a reagent or kit comprising the double-stranded RNAi agent or the DNA molecule or the vector.

The present invention also provides a cell comprising the above-mentioned double-stranded RNAi agent.

The present invention additionally provides a pharmaceutical composition comprising the above-mentioned double-stranded RNAi agent.

The pharmaceutical composition comprises a pharmacologically effective amount of the double-stranded RNAi agent of the present invention and other pharmaceutically acceptable components. "Effective amount" refers to a double-stranded RNAi dose that is effective in producing the expected pharmacological therapeutic effect. "Other components" include water, saline, glucose, buffers (such as PBS), excipients, diluents, disintegrants, binding agents, lubricants, sweeteners, flavoring agents, preservatives or combinations thereof.

The present invention also provides a method for inhibiting the expression of PCSK9 in a cell, which comprises: (a) contacting the cell with the above-mentioned double-stranded RNAi agent or the pharmaceutical composition thereof; (b) maintaining the cell produced in step (a) for a period of time that is sufficient to obtain degradation of the mRNA transcript of the PCSK9 gene, thereby inhibiting the expression of the PCSK9 gene in the cell.

The present invention provides use of the above-mentioned double-stranded RNAi agent or the pharmaceutical composition thereof in inhibiting PCSK9 gene expression or in manufacturing a product for inhibiting PCSK9 gene expression, wherein inhibiting PCSK9 gene expression is inhibiting or reducing the expression level of human, monkey, rat, or mouse PCSK9 gene in cells in vivo or in vitro. The cells are mammalian cells expressing PCSK9, such as primate cells and human cells. Preferably, the PCSK9 gene is expressed at a high level in the target cells. More preferably, the cells are derived from brain, salivary glands, heart, spleen, lungs, liver, kidneys, intestine, tumor. Further preferably, the cells are liver cancer cells, cervical cancer cells. Even more preferably, the cells are selected from HepG2, HEP3B, Huh7, COS7, 293T, MHCC97H, Hela, mouse primary hepatocytes, human primary hepatocytes. In some examples of the invention, the final cellular concentration of the double-stranded RNAi agent is 0.1-1000 nM, such as 10-500 nM, 25-300 nM or 50-100 nM.

In some examples of the invention, the double-stranded RNAi agent and the pharmaceutical composition thereof can be administered by any suitable means, such as parenteral administration and oral administration, including intramuscular, intravenous, arterial, peritoneal, or subcutaneous injection. Modes of administration include, but are not limited to, single administration or multiple administrations. The dosage range is 0.1 mg/kg to 100 mg/kg, 0.5 mg/kg to 50 mg/kg, 2.5 mg/kg to 20 mg/kg, 5 mg/kg to 15 mg/kg, specifically such as 3 mg/kg, 5 mg/kg, 10 mg/kg, 33 mg/kg.

The present invention also provides use of the double-stranded RNAi agent or the pharmaceutical composition thereof in reducing the concentrations of low-density lipoprotein (LDL) and/or low-density lipoprotein cholesterol (LDL-C) in serum or in the manufacture of a product for reducing the concentrations of low-density lipoprotein (LDL) and/or low-density lipoprotein cholesterol (LDL-C) in serum.

Wherein, reducing the concentrations of low-density lipoprotein (LDL) and/or low-density lipoprotein cholesterol (LDL-C) in the serum is reducing the concentrations of low-density lipoprotein (LDL) and/or low-density lipoprotein cholesterol (LDL-C) in the serum of humans, monkeys, rats or mice.

The concentration or content of LDL (low-density lipoprotein) or LDL-C (low-density lipoprotein cholesterol) in serum is reduced by at least 5%, 10%, 15%, 20%, 25%, 30%, 35%, 40%, 50%.

The present invention also provides use of the above-mentioned double-stranded RNAi agent or the pharmaceutical composition thereof in the manufacture of a medicament for preventing and/or treating a disease mediated by PCSK9 expression or alleviating a symptom of a disease mediated by PCSK9 expression.

The present invention also provides the above-mentioned double-stranded RNAi agent or the pharmaceutical composition thereof for use in preventing and/or treating a disease mediated by PCSK9 expression or alleviating a symptom of a disease mediated by PCSK9 expression.

The present invention also provides a method for preventing and/or treating a disease mediated by PCSK9 expression or alleviating a symptom of a disease mediated by PCSK9 expression, comprising administering to a subject in need thereof an effective amount of the double-stranded RNAi agent according to the present invention or the pharmaceutical composition thereof.

In the present invention, the disease mediated by PCSK9 expression includes a cardiovascular disease, dyslipidemia or neoplastic disease. The cardiovascular disease comprises atherosclerotic cardiovascular diseases, and dyslipidemia comprises elevated serum cholesterol and/or triglyceride levels, elevated low-density lipoprotein cholesterol, or elevated apolipoprotein B (ApoB), specifically such as mammalian hyperlipidemia, hypercholesterolemia, non-familial hypercholesterolemia, polygenic hypercholesterolemia, familial hypercholesterolemia, homozygous familial hypercholesterolemia or heterozygous familial hypercholesterolemia. The neoplastic disease includes PCSK9-related melanoma and metastatic liver cancer.

In some embodiments, a single dose of the pharmaceutical composition can be long-lasting, with the decrease in PCSK9 expression lasting at least 3, 5, 7, 10, 14 days or longer.

The innovation of the present invention is reflected in: 1. The high-throughput screened RNAi molecules have inhibitory activity comparable to or even higher than AD-60212 (positive control compound); 2. The modified RNAi molecules have high stability and high inhibitory activity. 3. While maintaining high inhibitory activity and stability, the ligand-modified RNAi molecules also have good liver targeting and the ability to promote cell endocytosis, which can reduce the impact on other tissues or organs, and reduce the usage amount of RNAi molecules, so that achieve the purpose of reducing toxicity and reducing costs; 4. Ligand-modified RNAi molecules can enter target cells and target tissues without transfection reagents, reducing the negative impact of transfection reagents, such as cells or tissue toxicity, thereby providing the possibility of targeted therapy; 5. Pharmacodynamic experiments in rhesus monkeys proved that compared with the positive control AD-60212, the candidate sequences reduced the levels of PCSK9 protein and LDL-c (low-density lipoprotein) by up to 90% and 65% respectively, and the effect on LDL-c was sustained for one week, and the effect was significant.

It should be noted that although many modifications can be attempted to improve the performance of double-stranded RNAi, these attempts are often difficult to elucidate both mediating RNA interference and having improved stability in serum (e.g., having increased resistance to nucleases and/or extended duration). The modified double-stranded RNAi of the present invention have high stability while maintaining high inhibitory activity.

### Definition

"PCSK9" refers to the proprotein convertase subtilisin Kexin9 gene or protein. PCSK9 is also known as FH3, HCHOLA3, NARC-1, or NARC1. Examples of PCSK9 mRNA sequences are readily available from, for example, GenBank.

"G", "C", "A" and "U" generally represent nucleotides containing guanine, cytosine, adenine and uracil as bases respectively. However, it is understood that the term "ribonucleotide" or "nucleotide" or "deoxyribonucleotide" may also refer to a modified nucleotide or an alternative replacement moiety. The skilled in the art will be well aware that guanine, cytosine, adenine and uracil can be substituted by other moieties without substantially changing base pairing properties of an oligonucleotide (including a nucleotide having such substituted moieties). Sequences comprising such substituted moieties are embodiments of the invention.

"RNAi agent" refers to an RNA transcript targeting cleavage agent that mediates the RNA-induced silencing complex (RISC) pathway. RNAi agents direct the sequence-specific degradation of mRNAs through a process known as RNA interference (RNAi). RNAi agents modulate, e.g., inhibit, the expression of PCSK9 in cells, such as cells in a subject (e.g., a mammalian subject).

In one embodiment, an RNAi agent of the invention includes a single-stranded RNA that interacts with a target RNA sequence, such as a PCSK9 target mRNA sequence, to direct cleavage of the target RNA.

In another embodiment, the RNAi agent can be a single-stranded siRNA introduced into a cell or organism to inhibit a target mRNA.

In another embodiment, the "RNAi agent" used in the composition, use, and method of the present invention is a double-stranded RNA, and is referred to herein as a "double-stranded RNAi agent." The term "double-stranded RNAi agent" refers to a complex of ribonucleic acid molecules, which has a double-stranded structure, comprises two antiparallel and substantially complementary nucleic acid strands, and is referred to as having "sense" and "antisense" orientations relative to the target RNA, i.e., the PCSK9 gene. In some embodiments of the invention, the double-stranded RNA triggers the degradation of target RNA, such as mRNA, through a post-transcriptional gene silencing mechanism (referred to herein as RNA interference or RNAi).

"Antisense strand" refers to the strand of a double-stranded RNAi agent that comprises a region that is substantially complementary to the target sequence (e.g., human PCSK9 mRNA).

"Sense strand" refers to an RNA strand comprising a region that is substantially complementary to a region of the antisense strand.

"Complementary region" refers to a region on the antisense strand that is completely or substantially complementary to the target mRNA sequence. In cases where the complementary region is not completely complementary to the target sequence, a mismatch can be located in the internal or terminal regions of the molecule. As used herein, the term "complementary" refers to the ability of a first polynucleotide to hybridize to a second polynucleotide under certain conditions, such as stringent conditions.

A "nucleotide overhang" refers to one or more unpaired nucleotides protruded from the duplex structure of an RNAi agent when the 3' end of one strand of the RNAi agent extends beyond the 5' end of the other strand, or vice versa. "Blunt end" or "blunt terminus" means that there is no unpaired nucleotide at that end of the double-stranded RNAi agent, i.e., no nucleotide overhang. A "blunt-ended" RNAi agent is a dsRNA that is double-stranded throughout its length, i.e., has no nucleotide overhang at either end of the molecule.

"Inhibiting PCSK9 expression in a cell" includes inhibiting the expression of any PCSK9 gene (e.g., mouse PCSK9 gene, rat PCSK9 gene, monkey PCSK9 gene, or human PCSK9 gene) as well as a variant (e.g., naturally occurring variant) or mutant of the PCSK9 gene. Thus, the PCSK9 gene may be a wild-type PCSK9 gene, a mutant PCSK9 gene, or in the case of a genetically manipulated cell, cell population, or organism, a transgenic PCSK9 gene.

The cells are mammalian cells expressing PCSK9, such as primate cells and human cells. Preferably, the PCSK9 gene is expressed at a high level in the target cells. More preferably, the cells are derived from the brain, salivary glands, heart, spleen, lungs, liver, kidneys, intestine, tumor. Further preferably, the cells are liver cancer cells or cervical cancer cells. Even more preferably, the cells are selected from HepG2, HEP3B, Huh7, COS7, 293T, MHCC97H, Hela, mouse primary hepatocytes, human primary hepatocytes.

"Inhibiting PCSK9 gene expression" includes any level of inhibition of the PCSK9 gene, such as at least partial inhibition of the expression of the PCSK9 gene, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75% , at least about 80%, at least about 85%, at least about 90%, at least about 91%, at least about 92%, at least about 93%, at least about 94%, at least about 95%, at least about 96%, at least about 97%, at least about 98%, or at least about 99%.

"Contacting a cell with a double-stranded RNAi agent" includes contacting the cell by any possible means. Contacting the cell with the double-stranded RNAi agent includes contacting the cell with the RNAi agent in vitro or contacting the cell with the RNAi agent in vivo. The contact can be made directly or indirectly. Thus, for example, the RNAi agent may come into physical contact with the cell through the individual performing the method, or alternatively, the RNAi agent may enter a situation that permits or causes it to subsequently come into contact with the cell.

"Disease mediated by PCSK9 expression" is intended to include any disease associated with the PCSK9 gene or protein. This disease may be caused, for example, by overproduction of the PCSK9 protein, by mutations in the PCSK9 gene, by abnormal cleavage of the PCSK9 protein, by abnormal interactions between PCSK9 and other proteins or other endogenous or exogenous substances.

"Hypercholesterolemia" refers to a condition characterized by elevated serum cholesterol. "Hyperlipidemia" refers to a condition characterized by elevated serum lipids. "Nonfamilial hypercholesterolemia" refers to a condition characterized by elevated cholesterol that is not caused by a single inherited gene mutation. "Polygenic hypercholesterolemia" refers to a condition characterized by elevated cholesterol caused by the influence of multiple genetic factors. "Familial hypercholesterolemia (FH)" refers to an autosomal dominant metabolic disorder characterized by mutations in the LDL-receptor (LDL-R), significantly elevated LDL-C, and premature onset of atherosclerosis. "Homozygous familial hypercholesterolemia (HoFH)" refers to a condition characterized by mutations in maternal and paternal LDL-R genes. "Heterozygous familial hypercholesterolemia (HoFH)" refers to a condition characterized by mutations in the maternal or paternal LDL-R gene.

A wide variety of entities can be conjugated to the RNAi agents of the invention. Preferred moieties are ligands that are covalently conjugated, preferably directly, or indirectly via an intervening tether.

Ligands may generally comprise therapeutic modifiers, for example to enhance uptake; diagnostic compounds or reporter groups, for example to monitor distribution; cross-linking agents; and moieties that confer nuclease resistance. General examples include lipids, steroids, vitamins, sugars, proteins, peptides, polyamines and peptidomimetics.

The ligand may include a naturally occurring substance such as a protein (e.g. human serum albumin (HSA), low density lipoprotein (LDL), high density lipoprotein (HDL) or globulin); a carbohydrate (e.g. a dextran, amylopectin, chitin, chitosan, inulin, cyclodextrin or hyaluronic acid); or a lipid. The ligand can also be a recombinant or synthetic molecule, such as a synthetic polymer, e.g. a synthetic polyamino acid, an oligonucleotide (e.g. an aptamer). Examples of polyamino acids include the following polyamino acids: polylysine (PLL), poly-L-aspartic acid, poly-L-glutamic acid, styrene-maleic anhydride copolymer, poly(L-lactide-co-glycolide) copolymer, divinyl ether-maleic anhydride copolymer, N-(2-hydroxypropyl)methacrylamide copolymer (HMPA), polyethylene glycol (PEG), polyvinyl alcohol ( PVA), polyurethane, poly(2-ethylacrylic acid), N-isopropylacrylamide polymer or polyphosphazine. Examples of polyamines include: polyethylenimine, polylysine (PLL), spermine, spermidine, polyamines, pseudopeptide-polyamines, peptidomimetic polyamines, dendrimer polyamines, arginine, amidine, protamine, cationic lipids, cationic porphyrins, quaternary salts of polyamines, or α-helical peptides.

Ligands may also include targeting groups, such as cell or tissue targeting agents that bind to a given cell type, such as kidney cells, such as lectins, glycoproteins, lipids, or proteins, such as antibodies. Targeting groups can be thyroid-stimulating hormone, melanocyte-stimulating hormone, lectins, glycoproteins, surfactant protein A, mucin carbohydrates, polyvalent lactose, polyvalent galactose, N-acetyl-galactosamine, N- acetyl-glucosamine, polyvalent mannose, polyvalent trehalose, glycosylated polyamino acids, polyvalent galactose, transferrin, bisphosphonates, polyglutamate, polyaspartate, lipids, cholesterol, steroids, cholic acid, folates, vitamin B12, biotin, RGD peptide, RGD peptide mimetics or aptamers.

Other examples of ligands include dyes, intercalators (e.g., acridine), cross-linkers (e.g., psoralen, mitomycin C), porphyrins (TPPC4, texaphyrin, Sapphyrin, polycyclic aromatic hydrocarbons (e.g., phenazine, dihydrophenazine), artificial endonucleases or chelating agents (e.g., EDTA), lipophilic molecules, such as cholesterol, cholic acid, adamantane acetic acid, 1- pyrenebutyric acid, dihydrotestosterone, 1,3-bis-O(hexadecyl)glycerol, geranyloxyhexyl, cetylglycerin, borneol, menthol, 1,3-propanediol, heptadecyl, palmitic acid, myristic acid, O3-(oleoyl)lithocholic acid, O3-(oleoyl)cholenoic acid, dimethoxytrityl, or phenoxazine peptide conjugates (e.g., antennapedia peptide, Tat peptide), alkylating agent, phosphate, amino, thiol, PEG (e.g., PEG-40K), MPEG, [MPEG]₂, polyamino, alkyl, substituted alkyl, radiolabeled markers, enzymes, haptens (e.g., biotin), transport/absorption enhancers (e.g., aspirin, vitamin E, folic acid), synthetic ribonucleases (e.g., imidazole, bisimidazole, histamine, imidazole clusters, acridine-imidazole conjugates, Eu³⁺ complexes of tetraazamacrocycles), dinitrophenyl, HRP or AP.

A ligand can be a protein, such as a glycoprotein, or a peptide, such as a molecule that has a specific affinity for a coligand, or an antibody, such as that binds to a given cell type (such as a cancer cell, an endothelial cell, or a bone cell). Ligands may also include hormones and hormone receptors. They may also include non-peptide species such as lipids, lectins, carbohydrates, vitamins, cofactors, polyvalent lactose, polyvalent galactose, N-acetyl-galactosamine, N-acetyl-glucosamine, polyvalent mannose, polyvalent fucose or aptamers. The ligand may be, for example, lipopolysaccharide, an activator of p38 MAP kinase or an activator of NF-κB.

The ligand can be a substance, e.g., a drug, that can increase the uptake of an iRNA agent into a cell, e.g., by disrupting the cell's cytoskeleton (e.g., by disrupting cellular microtubules, microfilaments, and/or intermediate filaments). Drugs may be, for example, taxon, vincristine, vinblastine, cytochalasin, nocodazole, japlakinolide, Latrunculin A, phalloidin, swinholide A, indanocine or myoservin.

A ligand can be any ligand capable of targeting a specific receptor. Examples are: folate, GalNAc, galactose, mannose, mannose-6P, sugar clusters (such as GalNAc clusters, mannose clusters, galactose clusters) or an aptamer. A cluster is a combination of two or more sugar units. These targeting ligands also include integrin receptor ligands, chemokine receptor ligands, transferrin, biotin, serotonin receptor ligands, PSMA, endothelin, GCPII, somatostatin, LDL, and HDL ligands. These ligands can also be based on nucleic acids, such as an aptamer. The aptamer can be unmodified or has any combination of modifications disclosed herein.

### Description of the drawings

Figure 1: DLR high-throughput screen of gene expression of PCSK9 candidate sequences.
Figure 2: Gene expression of Top16 candidate modified sequences in Hep3B cells.
Figure 3: Free uptake experiment of GalNAc-siRNA in human primary hepatocytes.
Figure 4: EC₅₀ values of GalNAc-siRNA candidate sequences in human primary hepatocytes.
Figure 5: IC₅₀ values of GalNAc-siRNA candidate sequences in HEP3B cells.
Figure 6: Binding abilities of different GalNAc-modified siRNAs to primary mouse hepatocytes.
Figure 7: In vivo liver targeting test of GalNAc-siRNAs.
Figure 8: In vivo drug efficacy test of GalNAc-siRNAs in mice.
Figure 9: Gene expression of Top2 candidate sequence modification strategies in Hep3B cells.
Figure 10: In vivo drug efficacy experiments in mice of 1168 sequence with different chemically modification.
Figure 11: Screening experiment for candidate sequences to reduce mouse PCSK9 protein level.
Figure 12A: Changes in LDLc levels caused by candidate sequences in cynomolgus monkeys.
Figure 12B: Changes in PCSK9 protein levels caused by candidate sequences in cynomolgus monkeys.
Figure 12C: PK distribution of candidate sequences in cynomolgus monkeys.
Figure 13: Toxicological studies of candidate sequences in mice.

### Detailed description

### Example 1. PCSK9-siRNA activity screening

### I. siRNA design

Based on human PCSK9 mRNA sequence, different sites were selected to design multiple PCSK9 siRNAs. All single siRNAs designed could target all transcripts of the target gene (as shown in Table 1). The above sequences (as shown in Table 2) were aligned by sequence similarity software, and had minimum homology to all other non-target gene sequences. For sequence design methods, please refer to the methods of Elbashir et al. 2002; Paddison et al. 2002; Reynoldset al. 2004; Ui-Tei et al. 2004 and others.

**Table 1 Target gene**

| Target gene | Species | Gene ID | NM_ID |
|---|---|---|---|
| PCSK9 | Homo sapiens | 255738 | NM_174936.3 |

**Table 2 High-throughput screened sequences**

| name | Sequence (5'->3') | length | Molecular weight (g/mol) | GC content |
|---|---|---|---|---|
| AD60212 | CUAGACCUGUUUUGCUUUUGU (SEQ ID NO:57) | 21 | 6565.89 | 38.1 |
| | ACAAAAGCAAAACAGGUCUAGAA (SEQ ID NO:58) | 23 | 7431.67 | 34.8 |
| U0041 | CUGGGGCUGAGCUUUAAAAUG (SEQ ID NO:59) | 21 | 6753.12 | 47.6 |
| | CAUUUUAAAGCUCAGCCCCAGCC (SEQ ID NO:60) | 23 | 7226.4 | 52.2 |
| U0044 | GGGCUGAGCUUUAAAAUGGUU (SEQ ID NO:61) | 21 | 6754.11 | 42.9 |
| | .AACCAUUUUAAAGCUCAGCCCCA (SEQ ID NO:62) | 23 | 7234.43 | 43.> |
| U0046 | GCUGAGCUUUAAAAUGGUUCC (SEQ ID NO:63) | 21 | 6674.05 | 42.9 |
| | GGAACCAUUUUAAAGCUCAGCCC (SEQ ID NO:64) | 23 | 7290.46 | 47.8 |
| U0142 | CCUGGCCCUUGAGUGGGGCAG (SEQ ID NO:65) | 21 | fi7>9.11 | 71.4 |
| | CUGCCCCACUCAAGGGCCAGGCC (SEQ ID NO:66) | 23 | 7295.46 | 73.9 |
| 10209 | GGAGGUGCCAGGAAGCUCCCU (SEQ ID NO:67) | 21 | 6766.15 | 66.7 |
| | AGGGAGCUUCCUGGCACCUCCAC (SEQ ID NO:68) | 23 | 7297.44 | 65.2 |
| U0210 | GAGGUGCCAGGAAGCUCCCUC (SEQ ID NO:69) | 21 | 6726.12 | 66.7 |
| | GAGGGAGCUUCCUGGCACCUCCA (SEQ ID NO:70) | 23 | 7337.47 | 65.2 |
| U0231 | CCUCACUGUGGGGCAUUUCAC (SEQ ID NO:71) | 21 | 6625 | 57.1 |
| | GUGAAAUGCCCCACAGUGAGGGA (SEQ ID NO:72) | 23 | 7448.6 | 56.5 |
| U0232 | CUCACUGUGGGGCAUUUCACC (SEQ ID NO:73) | 21 | 6625 | 57.1 |
| | GGUGAAAUGCCCCACAGUGAGGG (SEQ ID NO:74) | 23 | 7464.6 | 60.9 |
| U0237 | UGUGGGGCAUUUCACCAUUCA (SEQ ID NO:75) | 21 | 6650.02 | 47.6 |
| | UGAAUGGUGAAAUCCCCCACAGU (SEQ ID NO:76) | 23 | 7370.52 | 47.8 |
| U0322 | ACUUUUAUUGAGCUCUUGUUC (SEQ ID NO:77) | 21 | 6549.89 | 33.3 |
| | GAACAAGAGCUCAAUAAAAGUCA (SEQ ID NO:78) | 23 | 7408.63 | 34.8 |
| U0542 | GGCAUCUAGCCAGAGGCUGGA (SEQ ID NO:79) | 21 | 6790.18 | 61.9 |
| | UCCAGCCUCUGGCUAGAUGCCAU (SEQ ID NO:80) | 23 | 7259.39 | 56.5 |
| U0543 | GCAUCUAGCCAGAGGCUGGAG (SEQ ID NO:81) | 21 | 6790.18 | 61.9 |
| | CUCCAGCCUCUGGCUAGAUGCCA (SEQ ID NO:82) | 23 | 7258.4 | 60.9 |
| U0910 | GGUAACAGUGAGGCUGGGAAG (SEQ ID NO:83) | 21 | 6894.27 | 57.1 |
| | CUUCCCAGCCUCACUGUUACCCG (SEQ ID NO:84) | 23 | 7155.3 | 60.9 |
| U0920 | AGGCUGGGAAGGGGAACACAG (SEQ ID NO:85) | 21 | 6916.32 | 61.9 |
| | CUGUGUUCCCCUUCCCAGCCUCA (SEQ ID NO:86) | 23 | 7132.26 | 60.9 |
| U0926 | GGAAGGGGAACACAGACCAGG (SEQ ID NO:87) | 21 | 6899.33 | 61.9 |
| | CCUGGUCUGUGUUCCCCUUCCCA (SEQ ID NO:88) | 23 | 7149.25 | 60.9 |
| U0929 | AGGGGAACACAGACCAGGAAG (SEQ ID NO:89) | 21 | 6883.33 | 57.1 |
| | CUUCCUGGUCUGUGUUCCCCUUC (SEQ ID NO:90) | 23 | 7127.2 | 56.5 |
| U0930 | GGGGAACACAGACCAGGAAGC (SEQ ID NO:91) | 21 | 6859.3 | 61.9 |
| | GCUUCCUGGUCUGUGUUCCCCUU (SEQ ID NO:92) | 23 | 7167.23 | 56.5 |
| U0931 | GGGAACACAGACCAGGAAGCU (SEQ ID NO:93) | 21 | 6820.26 | 57.1 |
| | AGCUUCCUGGUCUGUGUUCCCCU (SEQ ID NO:94) | 23 | 7190.27 | 56.5 |
| U0932 | GGAACACAGACCAGGAAGCUC (SEQ ID NO:95) | 21 | 6780.23 | 57.1 |
| | GAGCUUCCUGGUCUGUGUUCCCC (SEQ ID NO:96) | 23 | 7229.31 | 60.9 |
| U1096 | ACCCAAGCAAGCAGACAUUUA (SEQ ID NO:97) | 21 | 6686.15 | 42.9 |
| | UAAAUGUCUGCUUGCUUGGGUGG (SEQ ID NO:98) | 23 | 7358.42 | 47.8 |
| U1097 | CCCAAGCAAGCAGACAUUUAU (SEQ ID NO:99) | 21 | 6663.11 | 42.9 |
| | AUAAAUGUCUGCUUGCUUGGGUG (SEQ ID NO:100) | 23 | 7342.42 | 43.5 |
| U1098 | CCAAGCAAGCAGACAUUUAUC (SEQ ID NO:101) | 21 | 6663.11 | 42.9 |
| | GAUAAAUGUCUGCUUGCUUGGGU (SEQ ID NO:102) | 23 | 7342.42 | 43.5 |
| U1099 | CAAGCAAGCAGACAUUUAUCU (SEQ ID NO:103) | 21 | 6664.1 | 38.1 |
| | AGAUAAAUGUCUGCUUGCUUGGG (SEQ ID NO:104) | 23 | 7365.46 | 43.5 |
| U1106 | GCAGACAUUUAUCUUUUGGGU (SEQ ID NO:105) | 21 | 6652 | 38.1 |
| | ACCCAAAAGAUAAAUGUCUGCUU (SEQ ID NO:106) | 23 | 7299.48 | 34.8 |
| U1120 | UUUGGGUCUGUCCUCUCUGUU (SEQ ID NO:107) | 21 | 6557.86 | 47.6 |
| | AACAGAGAGGACAGACCCAAAAG (SEQ ID NO:108) | 23 | 7485.72 | 47.8 |
| U1122 | UGGGUCUGUCCUCUCUGUUGC (SEQ ID NO:109) | 21 | 6595.91 | 57.1 |
| | GCAACAGAGAGGACAGACCCAAA (SEQ ID NO:110) | 23 | 7461.69 | 52.2 |
| U1123 | GGGUCUGUCCUC UCUGUUGCC (SEQ IDNO:111) | 21 | 6594.92 | 61.9 |
| | GGCAACAGAGAGGACAGACCCAA (SEQ ID NO:112) | 23 | 7477.69 | 56.5 |
| U1124 | GGUCUGUCCUCUCUGUUGCCU (SEQ ID NO:113) | 21 | 6555.88 | 57.1 |
| | AGGCAACAGAGAGGACAGACCCA (SEQ ID NO:114) | 23 | 7477.69 | 56.5 |
| U1125 | GUCUGUCCUCUCUGUUGCCUU (SEQ ID NO:115) | 21 | 6516.84 | 52.4 |
| | AAGGCAACAGAGAGGACAGACCC (SEQ ID NO:116) | 23 | 7477.69 | 56.5 |
| U1127 | CUGUCCUCUCUGUUGCCUUUU (SEQ ID NO:117) | 21 | 6477.8 | 47.6 |
| | AAAAGGCAACAGAGAGGACAGAC (SEQ ID NO:118) | 23 | 7525.75 | 47.8 |
| U1128 | UGUCCUCUCUGUUGCCUUUUU (SEQ ID NO:119) | 21 | 6478.79 | 42.9 |
| | AAAAAGGCAACAGAGAGGACAGA (SEQ ID NO:120) | 23 | 7549.78 | 43.5 |
| U1129 | GUCCUCUCUGUUGCCUUUUUA (SEQ ID NO:121) | 21 | 6501.83 | 42.9 |
| | UAAAAAGGCAACAGAGAGGACAG (SEQ ID NO:122) | 23 | 7526.74 | 43.5 |
| U1130 | UCCUCUCUGUUGCCUUUUUAC (SEQ ID NO:123) | 21 | 6461.8 | 42.9 |
| | GUAAAAAGGCAACAGAGAGGACA (SEQ ID NO:124) | 23 | 7526.74 | 43.5 |
| U1131 | CCUCUCUGUUGCCUUUUUACA (SEQ ID NO:125) | 21 | 6484.84 | 42.9 |
| | UGUAAAAAGGCAACAGAGAGGAC (SEQ ID NO:126) | 23 | 7503.7 | 43.5 |
| U1134 | CUCUGUUGCCUUUUUACAGCC (SEQ ID NO:127) | 21 | 6523.88 | 47.6 |
| | GGCUGUAAAAAGGCAACAGAGAG (SEQ ID NO:128) | 23 | 7519.7 | 47.8 |
| U1135 | UCUGUUGCCUUUUUACAGCCA (SEQ ID NO:129) | 21 | 6547.91 | 42.9 |
| | UGGCUGUAAAAAGGCAACAGAGA (SEQ ID NO:130) | 23 | 7480.66 | 43.5 |
| U1139 | UUGCCUUUUUACAGCCAACUU (SEQ ID NO:131) | 21 | 6531.91 | 38.1 |
| | AAGUUGGCUGUAAAAAGGCAACA (SEQ ID NO:132) | 23 | 7441.62 | 39.1 |
| U1164 | AGACCUGUUUUGCUUUUGUAA (SEQ ID NO:133) | 21 | 6612.96 | 33.3 |
| | UUACAAAAGCAAAACAGGUCUAG (SEQ ID NO:134) | 23 | 7385.59 | 34.8 |
| U1165 | GACCUGUUUUGCUUUUGUAAC (SEQ ID NO:135) | 21 | 6588.93 | 38.1 |
| | GUUACAAAAGCAAAACAGGUCUA (SEQ ID NO:136) | 23 | 7385.59 | 34.8 |
| U1166 | ACCUGUUUUGCUUUUGUAACU (SEQ ID NO:137) | 21 | 6549.89 | 33.3 |
| | AGUUACAAAAGCAAAACAGGUCU (SEQ ID NO:138) | 23 | 7385.59 | 34.8 |
| U1168 | CUGUUUUGCUUUUGUAACUUG (SEQ ID NO:139) | 21 | 6566.88 | 33.3 |
| | CAAGUUACAAAAGCAAAACAGGU (SEQ ID NO:140) | 23 | 7408.63 | 34.8 |
| U1169 | UGUUUUGCUUUUGUAACUUGA (SEQ ID NO:141) | 21 | 6590.91 | 28.6 |
| | UCAAGUUACAAAAGCAAAACAGG (SEQ ID NO:142) | 23 | 7408.63 | 34.8 |
| U1178 | UUUGUAACUUGAAGAUAUUUA (SEQ ID NO:143) | 21 | 6645.02 | 19 |
| | UAAAUAUCUUCAAGUUACAAAAG (SEQ ID NO:144) | 23 | 7308.5 | 21.7 |
| U1179 | UUGUAACUUGAAGAUAUUUAU (SEQ ID NO:145) | 21 | 6645.02 | 19 |
| | AUAAAUAUCUUCAAGUUACAAAA (SEQ ID NO:146) | 23 | 7292.5 | 17.4 |
| U1180 | UGUAACUUGAAGAUAUUUAUU (SEQ ID NO:147) | 21 | 6645.02 | 19 |
| | AAUAAAUAUCUUCAAGUUACAAA (SEQ ID NO:148) | 23 | 7292.5 | 17.4 |
| U1181 | GUAACUUGAAGAUAUUUAUUC (SEQ ID NO:149) | 21 | 6644.03 | 23.8 |
| | GAAUAAAUAUCUUCAAGUUACAA (SEQ ID NO:150) | 23 | 7308.5 | 21.7 |
| U1192 | AUAUUUAUUCUGGGUUUUGUA (SEQ ID NO:151) | 21 | 6614.94 | 23.8 |
| | UACAAAACCCAGAAUAAAUAUCU (SEQ ID NO:152) | 23 | 7290.52 | 26.1 |
| U1193 | UAUUUAUUCUGGGUUUUGUAG (SEQ ID NO:153) | 21 | 6630.94 | 28.6 |
| | CUACAAAACCCAGAAUAAAUAUC (SEQ ID NO:154) | 23 | 7289.53 | 30.4 |
| U1195 | UUUAUUCUGGGUUUUGUAGCA (SEQ ID NO:155) | 21 | 6629.95 | 33.3 |
| | UGCUACAAAACCCAGAAUAAAUA (SEQ ID NO:156) | 23 | 7329.56 | 30.4 |
| U1198 | AUUCUGGGUUUUGUAGCAUUU (SEQ ID NO:157) | 21 | 6629.95 | 33.3 |
| | AAAUGCUACAAAACCCAGAAUAA (SEQ ID NO:158) | 23 | 7352.6 | 30.4 |
| U1201 | CUGGGUUUUGUAGCAUUUUUA (SEQ ID NO:159) | 21 | 6629.95 | 33.3 |
| | UAAAAAUGCUACAAAACCCAGAA (SEQ ID NO:160) | 23 | 7352.6 | 30.4 |
| U1202 | UGGGUUUUGUAGCAUUUUUAU (SEQ ID NO:161) | 21 | 6630.94 | 28.6 |
| | AUAAAAAUGCUACAAAACCCAGA (SEQ ID NO:162) | 23 | 7352.6 | 30.4 |
| P598 | CCCGCCGGGGAUACCUCACCA (SEQ ID NO:163) | 21 | 6645.07 | 71.4 |
| | GUGAGGUAUCCCCGGCGGGCA (SEQ ID NO:164) | 21 | 6782.15 | 71.4 |
| P604 | GGGGAUACCUCACCAAGAUCC (SEQ ID NO:165) | 21 | 6694.12 | 57.1 |
| | AUCUUGGUGAGGUAUCCCCGG (SEQ ID NO:166) | 21 | 6705.06 | 57.1 |
| P605 | GGGAUACCUCACCAAGAUCCU (SEQ ID NO:167) | 21 | 6655.08 | 52.4 |
| | GAUCUUGGUGAGGUAUCCCCG (SEQ ID NO:168) | 21 | 6705.06 | 57.1 |
| P615 | ACCAAGAUCCUGCAUGUCUUC (SEQ ID NO:169) | 21 | 6593 | 47.6 |
| | AGACAUGCAGGAUCUUGGUGA (SEQ ID NO:170) | 21 | 6776.16 | 47.6 |
| P703 | CCCAUGUCGACUACAUCGAGG (SEQ ID NO:171) | 21 | 6671.08 | 57.1 |
| | UCGAUGUAGUCGACAUGGGGC (SEQ ID NO:172) | 21 | 6768.13 | 57.1 |
| P1348 | CCAACUUUGGCCGCUGUGUGG (SEQ ID NO:173) | 21 | 6681.03 | 61.9 |
| | ACACAGCGGCCAAAGUUGGUC (SEQ ID NO:174) | 21 | 6734.15 | 57.1 |
| P1359 | CGCUGUGUGGACCUCUUUGCC (SEQ ID NO:175) | 21 | 6617.96 | 61.9 |
| | CAAAGAGGUCCACACAGCGGC (SEQ ID NO:176) | 21 | 6756.2 | 61.9 |
| P1824 | CACAACGCUUUUGGGGGUGAG (SEQ ID NO:177) | 21 | 6768.13 | 57.1 |
| | CACCCCCAAAAGCGUUGUGGG (SEQ ID NO:178) | 21 | 6710.12 | 61.9 |
| P1825 | ACAACGCUUUUGGGGGUGAGG (SEQ ID NO:179) | 21 | 6808.16 | 57.1 |
| | UCACCCCCAAAAGCGUUGUGG (SEQ ID NO:180) | 21 | 6671.08 | 57.1 |
| P1826 | CAACGCUUUUGGGGGUGAGGG (SEQ ID NO:181) | 21 | 6824.16 | 61.9 |
| | CUCACCCCCAAAAGCGUUGUG (SEQ ID NO:182) | 21 | 6631.05 | 57.1 |
| P1840 | GUGAGGGUGUCUACGCCAUUG (SEQ ID NO:183) | 21 | 6745.09 | 57.1 |
| | AUGGCGUAGACACCCUCACCC (SEQ ID NO:184) | 21 | 6630.06 | 61.9 |
| P1841 | UGAGGGUGUCUACGCCAUUGC (SEQ ID NO:185) | 21 | 6705.06 | 57.1 |
| | AAUGGCGUAGACACCCUCACC (SEQ ID NO:186) | 21 | 6654.09 | 57.1 |
| P1842 | GAGGGUGUCUACGCCAUUGCC (SEQ ID NO:187) | 21 | 6704.07 | 61.9 |
| | CAAUGGCGUAGACACCCUCAC (SEQ ID NO:188) | 21 | 6654.09 | 57.1 |
| P1844 | GGGUGUCUACGCCAUUGCCAG (SEQ ID NO:189) | 21 | 6704.07 | 61.9 |
| | GGCAAUGGCGUAGACACCCUC (SEQ ID NO:190) | 21 | 6710.12 | 61.9 |
| P1845 | GGUGUCUACGCCAUUGCCAGG (SEQ ID NO:191) | 21 | 6704.07 | 61.9 |
| | UGGCAAUGGCGUAGACACCCU (SEQ ID NO:192) | 21 | 6711.11 | 57.1 |
| P1846 | GUGUCUACGCCAUUGCCAGGU (SEQ ID NO:193) | 21 | 6665.03 | 57.1 |
| | CUGGCAAUGGCGUAGACACCC (SEQ ID NO:194) | 21 | 6710.12 | 61.9 |
| P1847 | UGUCUACCJCCAUUGCCAGGUG (SEQ ID NO:195) | 21 | 6665.03 | 57.1 |
| | CCUGGCAAUGGCGUAGACACC (SEQ ID NO:196) | 21 | 6710.12 | 61.9 |
| P1850 | CUACGCCAUUGCCAGGUGCUG (SEQ ID NO:197) | 21 | 6664.04 | 61.9 |
| | GCACCUGGCAAUGGCGUAGAC (SEQ ID NO:198) | 21 | 6750.15 | 61.9 |
| P1851 | UACGCCAUUGCCAGGUGCUGC (SEQ ID NO:199) | 21 | 6664.04 | 61.9 |
| | AGCACCUGGCAAUGGCGUAGA (SEQ ID NO:200) | 21 | 6774.18 | 57.1 |
| P2006 | CACCCACAAGCCGCCUGUGCU (SEQ ID NO:201) | 21 | 6606.03 | 66.7 |
| | CACAGGCGGCUUGUGGGUGCC (SEQ ID NO:202) | 21 | 6759.11 | 71.4 |
| P2008 | CCCACAAGCCGCCUGUGCUGA (SEQ ID NO:203) | 21 | 6646.06 | 66.7 |
| | AGCACAGGCGGCUUGUGGGUG (SEQ ID NO:204) | 21 | 6823.17 | 66.7 |
| P2009 | CCACAAGCCGCCUGUGCUGAG (SEQ ID NO:205) | 21 | 6686.09 | 66.7 |
| | CAGCACAGGCGGCUUGUGGGU (SEQ ID NO:206) | 21 | 6783.14 | 66.7 |
| P2032 | CACGAGGUCAGCCCAACCAGU (SEQ ID NO:207) | 21 | 6693.13 | 61.9 |
| | UGGUUGGGCUGACCUCGUGGC (SEQ ID NO:208) | 21 | 6737.06 | 66.7 |
| P2034 | CGAGGUCAGCCCAACCAGUGC (SEQ ID NO:209) | 21 | 6709.13 | 66.7 |
| | ACUGGUUGGGCUGACCUCGUG (SEQ ID NO:210) | 21 | 6721.06 | 61.9 |
| P2035 | GAGGUCAGCCCAACCAGUGCG (SEQ ID NO:211) | 21 | 6749.16 | 66.7 |
| | CACUGGUUGGGCUGACCUCGU (SEQ ID NO:212) | 21 | 6681.03 | 61.9 |
| P2256 | GUCAGGAGCCGGGACGUCAGC (SEQ ID NO:213) | 21 | 6805.19 | 71.4 |
| | UGACGUCCCGGCUCCUGACUA (SEQ ID NO:214) | 21 | 6624.01 | 61.9 |
| mus1076 | CGGCACCCUCAUAGGCCUGGA (SEQ ID NO:215) | 21 | 6686.09 | 66.7 |
| | UCCAGGCCUAUGAGGGUGCCGCU (SEQ ID NO:216) | 23 | 7354.46 | 65.2 |
| mus1078 | GCACCCUCAUAGGCCUGGAGU (SEQ ID NO:217) | 21 | 6687.08 | 61.9 |
| | ACUCCAGGCCUAUGAGGGUGCCG (SEQ ID NO:218) | 23 | 7377.5 | 65.2 |
| mus1080 | ACCCUCAUAGGCCUGGAGUUU (SEQ ID NO:219) | 21 | 6649.03 | 52.4 |
| | AAACUCCAGGCCUAUGAGGGUGC (SEQ ID NO:220) | 23 | 7385.53 | 56.5 |
| mus1082 | CCUCAUAGGCCUGGAGUUUAU (SEQ ID NO:221) | 21 | 6650.02 | 47.6 |
| | AUAAACUCCAGGCCUAUGAGGGU (SEQ ID NO:222) | 23 | 7370.52 | 47.8 |
| mus1084 | UCAUAGGCCUGGAGUUUAUUC (SEQ ID NO:223) | 21 | 6651.01 | 42.9 |
| | GAAUAAACUCCAGGCCUAUGAGG (SEQ ID NO:224) | 23 | 7393.56 | 47.8 |
| mus1086 | AUAGGCCUGGAGUUUAUUCGG (SEQ ID NO:225) | 21 | 6730.08 | 47.6 |
| | CCGAAUAAACUCCAGGCCUAUGA (SEQ ID NO:226) | 23 | 7313.5 | 47.8 |
| mus1088 | AGGCCUGGAGUUUAUUCGGAA (SEQ ID NO:227) | 21 | 6753.12 | 47.6 |
| | UUCCGAAUAAACUCCAGGCCUAU (SEQ ID NO:228) | 23 | 7251.42 | 43.5 |
| mus1448 | ACAGGCUGCUGCCCACGUGGC (SEQ ID NO:229) | 21 | 6702.09 | 71.4 |
| | GCCACGUGGGCAGCAGCCUGUGA (SEQ ID NO:230) | 23 | 7416.54 | 69.6 |
| mus1596 | CUGACCCCCAACCUGGUGGCC (SEQ ID NO:231) | 21 | 6622.03 | 71.4 |
| | GGCCACCAGGUUGGGGGUCAGUA (SEQ ID NO:232) | 23 | 7457.56 | 65.2 |
| mus1733 | GGAGCUGCUGAGCUGCUCCAC (SEQ ID NO:233) | 21 | 6743.11 | 66.7 |
| | CUGGAGCAGCUCAGCAGCUCCUC (SEQ ID NO:234) | 23 | 7297.44 | 65.2 |
| mus2084 | UUCCUGCUGCCAUGCCCCAGG (SEQ ID NO:235) | 21 | 6599.98 | 66.7 |
| | CCUGGGGCAUGGCAGCAGGAAGC (SEQ ID NO:236) | 23 | 7479.61 | 69.6 |
| mus2317 | CCAUCUGCUGCCGGAGCCGGC (SEQ ID NO:237) | 21 | 6678.06 | 76.2 |
| | GCCGGCUCCGGCAGCAGAUGGCA (SEQ ID NO:238) | 23 | 7415.55 | 73.9 |

### II. siRNA synthesis (natural RNA/2'-methoxy or 2'-fluoro modified RNA/GalNAc-RNA)

The oligonucleotides containing only ribonucleotides or 2'-methoxy or 2'-fluoro modified oligonucleotides of the present invention were synthesized according to the specification of the theoretical yield of 1 µmol. All oligonucleotides were prepared on a LK-192X synthesizer using 1 µmol of the universal Frit carrier (1000Å=100nm, Biocomma) or GalNAc CPG (WuXi AppTec Co., Ltd./Glen research, loading capacity 30umol/g). All phosphoramidite monomers (Hongene Biotech) were diluted with anhydrous acetonitrile solvent at 1:20 (g/mL), and the coupling time was 3 min, with a total of two couplings. 3% TCA was used for deprotection, and 0.3M benzylthiotetrazole acetonitrile solution was used for activation, and capping and oxidation were performed with CAPA/CAPB and 50 mM I₂ solutions respectively. After trityl-off synthesis, the solid phase carrier was transferred to a 2 mL centrifuge tube, added with 1.2 mL ammonia water, and heated in a 65°C oven for 3 hours to remove the protecting groups. After cooled to room temperature and vacuum concentrated for 30 minutes, the solution was filtered through a 0.22um filter membrane into a loading bottle. A semi-preparative reverse phase purification instrument was used for single-chain purification with an elution gradient of 7% to 30% (ACN: 100mM TEAA), time of 10 minutes, and flow rate of 5mL/min. The sample was vacuum concentrated after purification, spun to dry at room temperature, and was finally dissolved in water. Each solution was desalted on a GE Hi-Trap desalting column to elute the final oligonucleotide product. All properties and purity were confirmed using ESI-MS and IEX HPLC respectively. A microplate reader was used to determine the concentration using UV light. Equal molar amounts of sense strands and antisense strands were mixed into a new centrifuge tube. After heated at 95°C for 5 minutes and slowly annealed to room temperature, and finally spun to dry by a vacuum concentrator at room temperature, the final product was obtained.

### III. Detection of Inhibitory activity of PCSK9-siRNA in psicheck-2 system in vitro

### 1. Construct of detection plasmid

The psicheck-2 plasmid was used to construct a recombinant plasmid (Shanghai Generay Bioengineering Co., Ltd.), which comprised the target sequence of all PCSK9 siRNAs to be tested, and the cloning sites were the 5 'Xhol and 3 'NotI sites of the psicheck-2 plasmid.

### 2. Co-transfection of PCSK9 siRNA and the recombinant plasmid into different cells

All cells were purchased from the Cell Bank of the Chinese Academy of Sciences; other reagents were commercially available.

**Table 3 Cell names and types**

| Cell name | Cos7 | 293T | Hep3B |
|---|---|---|---|
| Cell type | liver cancer cell | liver cancer cell | liver cancer cell |

The cells were cultured in DMEM medium containing 10% fetal bovine serum in a 5% CO₂, 37°C constant-temperature incubator. When the cells were in the logarithmic growth phase and in good condition (70% confluence), they were plated and transfected. The cell density was adjusted, and the cells were plated into a 24-well plate at 1.5×10⁵ cells/well. The transfection complex was prepared as follows: 250 µL Opti-MEM, 40 ng recombinant plasmid and 5 µL 10 nM siRNA were mixed. 250 µL Opti-MEM and 2.5 µL Lipofectamine 2000 transfection reagent were mixed. After stood for 5 minutes, the above two mixtures were mixed and stood for 20 min. The above transfection complex was added into a 24-well plate and incubated in a 5% CO₂, 37°C constant-temperature incubator for 6 h. The supernatant was aspirated, and 1 mL of complete culture medium was added to each well and the culture was cultivated for another 24h.

In addition to the test group for each cell transfection, the following control groups were also set: NC was the negative control (irrelevant siRNA), Lipo group was the transfection reagent control group, and Blank group was the untreated control group (no siRNA was added). Both the test group and the control group were repeated three times.

### 3. DLR detection and analysis

The Dual-Luciferase Reporter Assay System kit (Promega) was used for detection. The cells were lysed and collected according to the instructions of the kit. The Infinite Eplex microplate reader (TECAN) was used to detect fluorescence intensity of Photinus pyralis luciferase and Renilla reniformis luciferase in sequence. The ratio of the fluorescence intensity of Renilla reniformis luciferase and that of Photinus pyralis luciferase was calculated, and NC group was used as the control for normalization. Table 4 shows the results of DLR detection, i.e., the average expression level of the dual-luciferase reporter gene in the PCSK9 siRNA test group relative to the NC group.

**Table 4 Results of DLR test**

| Number | 293T-01 | 293T-02 | COS.7-01 | COS.7-02 | average |
|---|---|---|---|---|---|
| U1128 | 0.2741 | 0.1768 | 0.4016 | 0.4992 | 0.3379 |
| U1129 | 0.2375 | 0.2266 | 0.5082 | 0.5239 | 0.3741 |
| U1164 | 0.1032 | 0.1090 | 0.1519 | 0.2200 | 0.1460 |
| U1165 | 0.1339 | 0.1347 | 0.2311 | 0.3754 | 0.2188 |
| U1166 | 0.1121 | 0.0831 | 0.1529 | 0.3702 | 0.1796 |
| U1168 | 0.2384 | 0.0948 | 0.2239 | 0.1471 | 0.1761 |
| U1178 | 0.2235 | 0.1579 | 0.3495 | 0.3638 | 0.2737 |
| U1179 | 0.1220 | 0.0787 | 0.1554 | 0.2810 | 0.1593 |
| U1180 | 0.1729 | 0.1370 | 0.1666 | 0.3562 | 0.2082 |
| U1181 | 0.3573 | 0.2243 | 0.3162 | 0.4211 | 0.3297 |
| U1192 | 0.4567 | 0.3359 | 0.2006 | 0.2764 | 0.3174 |
| U1195 | 0.3495 | 0.1332 | 0.1928 | 0.2581 | 0.2334 |
| U1198 | 0.2596 | 0.1193 | 0.1407 | 0.1836 | 0.1758 |
| U1201 | 0.0937 | 0.0534 | 0.1363 | 0.2310 | 0.1286 |
| U1202 | 0.1146 | 0.0656 | 0.1850 | 0.3287 | 0.1735 |
| U0044 | 0.1095 | 0.0775 | 0.1868 | 1.0693 | 0.3608 |

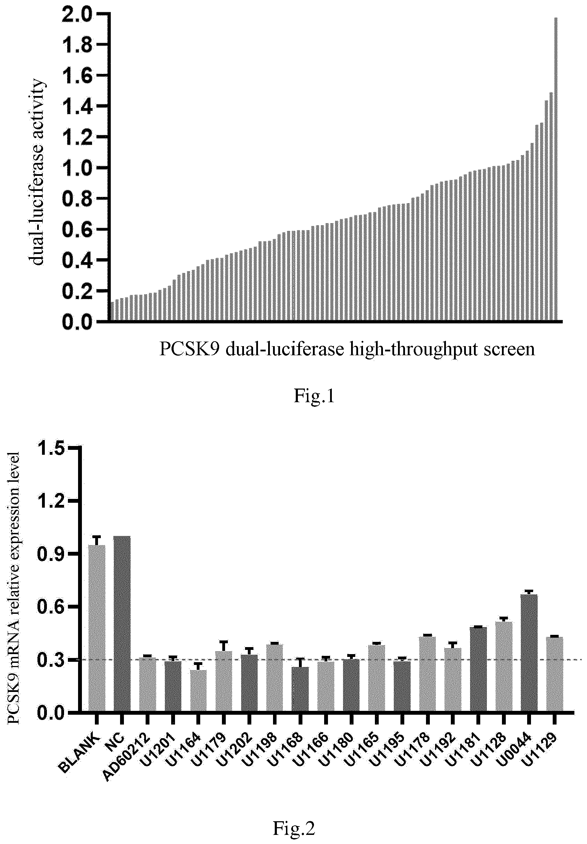

As shown in Figure 1, PCSK9 siRNA DLR screening in COS7 and 293T cells revealed the top 16 siRNA molecules with higher cell activity, which were homologous to those of human, cynomolgus monkey, and rhesus monkey. The sense strands of the top 16 siRNA molecules with higher cell activity were directed to positions between 3567-3663 or 2483-2505 of PCSK9, and the nucleotide sequences thereof were as shown in SEQ ID NO: 1 and SEQ ID NO respectively:

TCTGTCCTCTCTGTTGCCTTTTACAGCCAACTTTCTAGACCTGTTTGCTTTTGTAAC
TTGAAGATATTTATTCTGGGTTTTGTAGCATTTTTAT(SEQ ID NO:1)
TGGGGCTGAGCTTTAAAATGGTT(SEQ ID NO:2).

### IV. PCSK9 siRNA qPCR screening

### 1. transfecting Hep3B cells with PCSK9 siRNA

Hep3B cells were cultured in DMEM medium containing 10% fetal bovine serum in a 5% CO₂, 37°C constant-temperature incubator. When the cells were in the logarithmic growth phase and in good condition (70% confluence), they were plated and transfected. The cell density was adjusted, and the cells were plated into a 24-well plate at 1.5×10⁵ cells/well. The transfection complex was prepared as follows: 250 µL Opti-MEM and 5 µL 10 nM siRNA were mixed. 250 µL Opti-MEM and 2.5 µL Lipofectamine 2000 transfection reagent were mixed. After stood for 5 min, the above two mixtures were mixed and stood for 20 min. The above transfection complex was added into a 24-well plate and incubated in a 5% CO₂, 37°C constant-temperature incubator for 6 h. The supernatant was aspirated, and 1 mL of complete culture medium was added to each well and the culture was continued for 24h. In addition to the test group for each cell transfection, the following control groups were also set: NC was the negative control (irrelevant siRNA), Lipo group was the transfection reagent control group, and Blank group was the untreated control group (no siRNA was added).

### 2. Real-time fluorescence quantitative PCR analysis:

Cells were lysed 24 hours after transfection, and total cellular RNA was extracted using a column extraction kit (Norvizan). Using β-actin gene as the internal reference gene, Taqman probe method was used to perform real-time fluorescence quantitative PCR reaction using CFX96 fluorescence quantitative PCR instrument (Bio-Rad). The primers used were:

**Table 5 information on primer sequences**

| Name of primer | Sequence (5' to 3') |
|---|---|
| H-ACTB-FO-3 | TGCCGACAGGATGCAGAAG(SEQ ID NO:239) |
| H-ACTB-RE-3 | GCCGATCCACACGGAGTACT(SEQ ID NO:240) |
| H-ACTB-PR-3-FAM | ATCAAGATCATTGCTCCTCCTGAGCGC(SEQ ID NO:241) |
| H-PCSK9V1-FO-1 | GATCCTGCATGTCTTCCA(SEQ ID NO:242) |
| H-PCSK9V1-RE-1 | GTCCTCCTCGATGTAGTC(SEQ ID NO:243) |
| H-PCSK9V1-PR1-1-JOE | CCTTCTTCCTGGCTTCCTGGT(SEQ ID NO:244) |

### 3. Data analysis

After the PCR reaction, the 2-ΔΔCt (Livak) method was used to perform relative quantitative analysis using the reference gene as the standard. The results in Table 6 and Figure 2 show that 0.1 nM of U1164 and U1168 among the top 16 pairs of siRNA sequences had higher inhibitory activity in Hep3B cells, which were 23.2% and 17.5% higher than that of the positive control AD60212 respectively.

**Table 6 Inhibitory activities of the top 16 pairs of siRNA sequences**

| Relative expression | |
|---|---|
| Blank | 94.8% |
| NC | 100.0% |
| AD60212 | 31.4% |
| U1201 | 29.2% |
| U1164 | 24.1% |
| U1179 | 34.9% |
| U1202 | 32.9% |
| U1198 | 38.6% |
| U1168 | 25.9% |
| U1166 | 28.9% |
| U1180 | 30.3% |
| U1165 | 38.4% |
| U1195 | 29.1% |
| U1178 | 42.9% |
| U1192 | 36.6% |
| U1181 | 48.4% |
| U1128 | 51.5% |
| U0044 | 66.9% |
| U1129 | 43.0% |

### Example 2. Optimization of PCSK9-siRNAs

### I. Evaluation of the activities of GalNAc-siRNAs in inhibiting PCSK9 by free uptake of human primary hepatocytes

In order to further confirm the top 16 pairs of highly active siRNA molecules, we performed sequence modification and optimization on them respectively (Table 7). The synthesis steps were the same as in Example 1, and their inhibitory activities were evaluated by free uptake of human primary hepatocytes.

**Table 7 The top 16 highly active modified siRNA sequences**

| name | Sequence (5'->3') | length | Molecular weight (g/mole) | GC content |
|---|---|---|---|---|
| E1128 | UfsGmsUfCmCfUmCfUmCfUfGfUmUfGmCfCmUfUmUfU mUf-L96(SEQ ID NO:3) | 21 | 8449.77 | 42.9 |
| | AmsAfsAmAfAmGfGmCfAmAfCmAmGmAfGmAfGmGf AmCfAmsGmsAm(SEQ ID NO:4) | 23 | 7827.78 | 43.5 |
| E1129 | GfsUmsCfCmUfCmUfCmUfGfUfUmGfCmCfUmUfUmUfU mAf-L96(SEQ ID NO:5) | 21 | 8472.81 | 42.9 |
| | UmsAfsAmAfAmAfGmGfCmAfAmCmAmGfAmGfAmGf GmAfCmsAmsGm(SEQ ID NO:6) | 23 | 7804.74 | 43.5 |
| E1164 | AfsGmsAfCmCfUmGfUmUfUfUfGmCfUmUfUmUfGmUf AmAf-L96(SEQ ID NO:7) | 21 | 8583.94 | 33.3 |
| | UmsUfsAmCfAmAfAmAfGmCfAmAmAmAfCmAfGmGfU mCfUmsAmsGm(SEQ ID NO:8) | 23 | 7663.59 | 34.8 |
| E1165 | GfsAmsCfCmUfGmUfUmUfUfGfCmUfUmUfUmGfUmAf AmCf-L96(SEQ ID NO:9) | 21 | 8559.91 | 38.1 |
| | GmsUfsUmAfCmAfAmAfAmGfCmAmAmAfAmCfAmGfG mUfCmsUmsAm(SEQ ID NO:10) | 23 | 7663.59 | 34.8 |
| E1166 | AfsCmsCfUmGfUmUfUmUfGfCfUmUfUmUfGmUfAmAf CmUf-L96(SEQ ID NO:11) | 21 | 8520.87 | 33.3 |
| | AmsGfsUmUfAmCfAmAfAmAfGmCmAmAfAmAfCmAfG mGfUmsCmsUm(SEQ ID NO:12) | 23 | 7663.59 | 34.8 |
| E1168 | CfsUmsGfUmUfUmUfGmCfUfUfUmUfGmUfAmAfCmUf UmGf-L96(SEQ ID NO:13) | 21 | 8537.86 | 33.3 |
| | CmsAfsAmGfUmUfAmCfAmAfAmAmGmCfAmAfAmAfC mAfGmsGmsUm(SEQ ID NO:14) | 23 | 7686.63 | 34.8 |
| E1178 | UfsUmsUfGmUfAmAfCmUfUfGfAmAfGmAfUmAfUmUf UmAf-L96(SEQ ID NO:15) | 21 | 8616 | 19 |
| | UmsAfsAmAfUmAfUmCfUmUfCmAmAmGfUmUfAmCfA mAfAmsAmsGm(SEQ ID NO:16) | 23 | 7586.5 | 21.7 |
| E1179 | UfsUmsGfUmAfAmCfUmUfGfAfAmGfAmUfAmUfUmUf AmUf-L96(SEQ ID NO:17) | 21 | 8616 | 19 |
| | AmsUfsAmAfAmUfAmUfCmUfUmCmAmAfGmUfUmAfC mAfAmsAmsAm(SEQ ID NO:18) | 23 | 7570.5 | 17.4 |
| E1180 | UfsGmsUfAmAfCmUfUmGfAfAfGmAfUmAfUmUfUmAf UmUf-L96(SEQ ID NO:19) | 21 | 8616 | 19 |
| | AmsAfsUmAfAmAfUmAfUmCfUmUmCmAfAmGfUmUf AmCfAmsAmsAm(SEQ ID NO:20) | 23 | 7570.5 | 17.4 |
| E1181 | GfsUmsAfAmCfUmUfGmAfAfGfAmUfAmUfUmUfAmUf UmCf-L96(SEQ ID NO:21) | 21 | 8615.01 | 23.8 |
| | GmsAfsAmUfAmAfAmUfAmUfCmUmUmCfAmAfGmUf UmAfCmsAmsAm(SEQ ID NO:22) | 23 | 7586.5 | 21.7 |
| E1192 | AfsUmsAfUmUfUmAfUmUfCfUfGmGfGmUfUmUfUmGf UmAf-L96(SEQ ID NO:23) | 21 | 8585.92 | 23.8 |
| | UmsAfsCmAfAmAfAmCfCmCfAmGmAmAfUmAfAmAfU mAfUmsCmsUm(SEQ ID NO:24) | 23 | 7568.52 | 26.1 |
| E1195 | UfsUmsUfAmUfUmCfUmGfGfGfUmUfUmUfGmUfAmGf CmAf-L96(SEQ ID NO:25) | 21 | 8600.93 | 33.3 |
| | UmsGfsCmUfAmCfAmAfAmAfCmCmCmAfGmAfAmUfA mAfAmsUmsAm(SEQ ID NO:26) | 23 | 7607.56 | 30.4 |
| E1198 | AfsUmsUfCmUfGmGfGmUfUfUfUmGfUmAfGmCfAmUf UmUf-L96(SEQ ID NO:27) | 21 | 8600.93 | 33.3 |
| | AmsAfsAmUfGmCfUmAfCmAfAmAmAmCfCmCfAmGfA mAfUmsAmsAm(SEQ ID NO:28) | 23 | 7630.6 | 30.4 |
| E1201 | CfsUmsGfGmGfUmUfUmUfGfUfAmGfCmAfUmUfUmUf UmAf-L96(SEQ ID NO:29) | 21 | 8600.93 | 33.3 |
| | UmsAfsAmAfAmAfUmGfCmUfAmCmAmAfAmAfCmCfC mAfGmsAmsAm(SEQ ID NO:30) | 23 | 7630.6 | 30.4 |
| E1202 | UfsGmsGfGmUfUmUfUmGfUfAfGmCfAmUfUmUfUmUf AmUf-L96(SEQ ID NO:31) | 21 | 8601.92 | 28.6 |
| | AmsUfsAmAfAmAfAmUfGmCfUmAmCmAfAmAfAmCfC mCfAmsGmsAm(SEQ ID NO:32) | 23 | 7630.6 | 30.4 |
| E0044 | GfsGmsGfCmUfGmAfGmCfUfUfUmAfAmAfAmUfGmGf UmUf-L96(SEQ ID NO:33) | 21 | 8725.09 | 42.9 |
| | AmsAfsCmCfAmUfUmUfUmAfAmAmGmCfUmCfAmGfC mCfCmsCmsAm(SEQ ID NO:34) | 23 | 7512.43 | 43.5 |

In the above table, Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone. The structure of L96 is shown in formula IV:

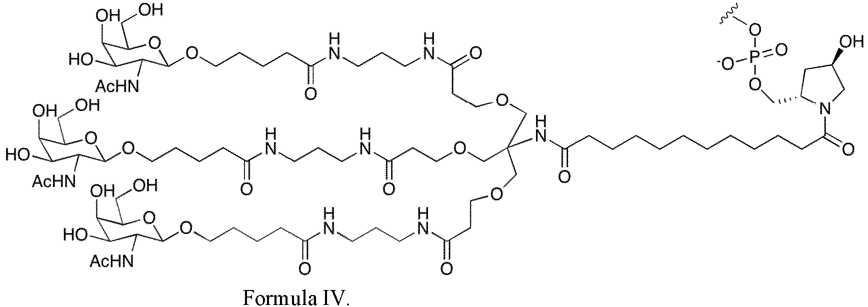

### 1. Inhibitory activities of 16 pairs of modified GalNAc-siRNA sequences on the free uptake of PCSK9 by human primary hepatocytes

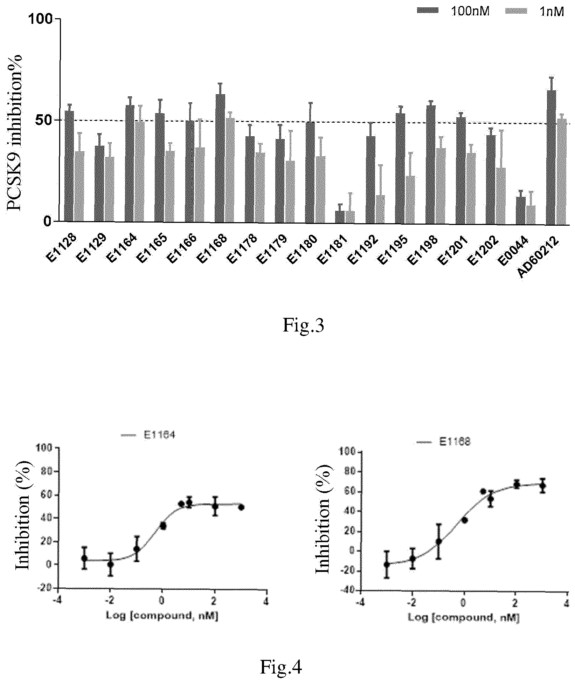

Frozen preserved human primary hepatocytes (purchased from the Cell Bank of the Chinese Academy of Sciences) were resuscitated and adjusted to an appropriate density, and then plated into a 96-well plate. GalNAc-siRNAs were diluted with PBS to a concentration of 100 nM or 1 nM. Then, a certain volume of diluted GalNAc-siRNAs was added to a 96-well plate and co-incubated with human primary hepatocytes for 48 hours. The supernatant was aspirated, and the cells were collected. Total cellular RNA was extracted with a column extraction kit to detect the relative expression of PCSK9 mRNA by qPCR. It can be seen from the results (Figure 3) that GalNAc-siRNAs could enter liver cells through endocytosis and silence the expression of PCSK9 gene, and E1164 and E1168 had higher inhibitory activities.

### 2. EC₅₀ value for free uptake by human primary hepatocytes

Human primary hepatocytes were adjusted to an appropriate density and plated into a 96-well plate. E1164 and E1168 were gradiently diluted into different concentrations with PBS, with the highest final concentration set at 100nM, and 10-fold gradient dilutions were made to obtain a total of 8 concentrations. Then a certain volume of E1164 and E1168 with different concentrations was taken and added into a 96-well plate, co-incubated with human primary liver cells for a total of 48 hours. The supernatant was aspirated, and the cells were collected. Total cellular RNA was extracted with a column extraction kit to detect the relative expression of PCSK9 mRNA by qPCR. EC₅₀ values were analyzed and calculated by Graphpad Prism software. As shown in Figure 4, the EC₅₀ values of E1164 and E1168 in human primary hepatocytes were 5.66nM and 3.45nM respectively.

### II IC₅₀ values for transfecting Hep3B cells

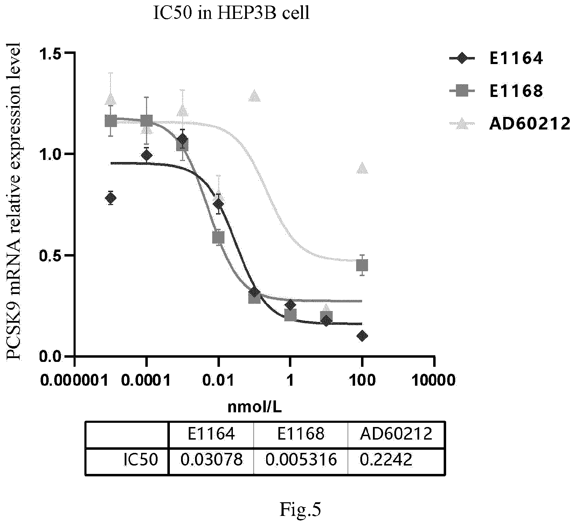

E1164, E1168 and positive control AD60212 were gradiently diluted to different concentrations with Nuclease-Free Water (Invitrogen), with the highest final concentration set at 100 nM, and 10-fold gradient dilutions were made to obtain a total of 8 concentrations to be used for transfecting Hep3B cells (purchased from the Cell Bank of the Chinese Academy of Sciences). For the steps of transfection and quantitative PCR detection and analysis, please refer to Example 1. IC₅₀ values were analyzed and calculated by Graphpad Prism software. As shown in Figure 5, the IC₅₀ values of E1164, E1168 and positive control AD60212 in Hep3B cells were 0.031nM, 0.005nM and 0.224nM respectively. The IC₅₀ values of E1164 and E1168 in Hep3B cells were 1/10 or 1/100 of that of the positive control AD60212, which were significantly better than the positive control AD60212.

### Example 3: Screening of GalNAc targeting structures

### I. Isolation of primary mouse liver cells

The mouse was anesthetized, and the skin and muscle layers thereof were cut to expose the liver, in which a perfusion catheter was inserted into the portal vein. A small opening was cut in the inferior vena cava to prepare the liver for perfusion. Perfusion Solution I (Hank's, 0.5mM EGTA, pH 8) and perfusion Solution II (Low-glucose DMEM, 100U/mL Type IV, pH7.4) were preheated at 40°C. Perfusion Solution I at 37°C was infused into the liver via the portal vein with the flow rate of 7mL/min for 5 minutes until the liver turned to gray-white. Then perfusion Solution II at 37°C was infused into the liver with the flow rate of 7mL/min for 7 minutes. After the perfusion was completed, the liver was removed and placed in Solution III (10% FBS low-glucose DMEM, 4°C) to terminate digestion. The liver capsule was scratched by forceps, and the liver was shaken gently to release hepatocytes. The liver cells were filtered through a 70 µm cell filter, and centrifuged at 50g for 2 minutes. Then, the supernatant was discarded. The cells were resuspended in Solution IV (40% percoll low-glucose DMEM, 4°C), centrifuged at 100g for 2 minutes. Then, the supernatant was discarded. 2% FBS low-glucose DMEM was added to resuspend the cells, which were set aside. Trypan blue staining was used to identify cell viability.

### II. Determination of GalNAc binding curve and K_{d} value

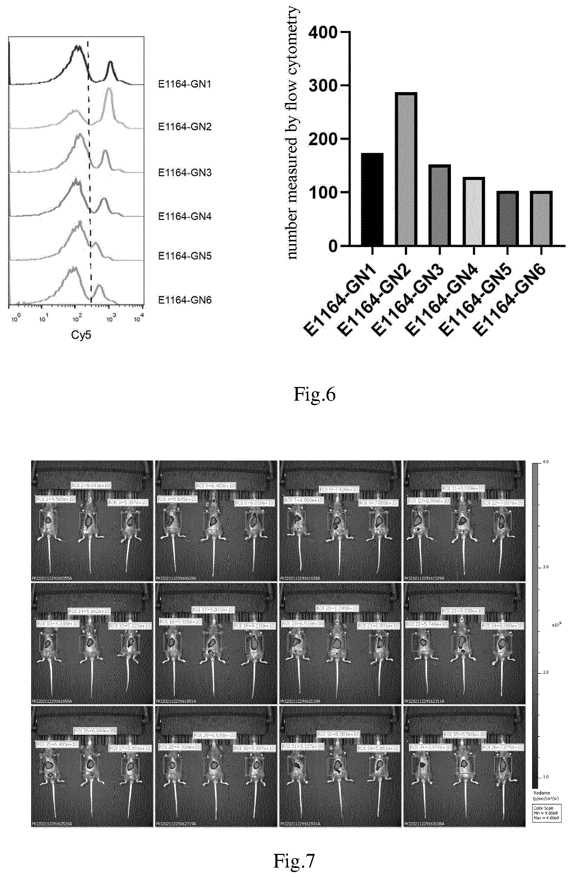

Freshly isolated mouse primary hepatocytes were plated into a 96-well plate at 2×10⁴ cells/well, 100 µl/well. GalNAc-siRNAs were added to each well (see Table 8). The final concentration of each GalNAc-siRNA was set to 20 nM. After the cells were incubated at 4°C for 2 hours and centrifuged at 50g for 2 minutes, the supernatant was discarded. The cells were resuspended with 10 µg/ml PI, stained for 10 min, and centrifuged at 50g for 2 min. The cells were washed with precooled PBS, and centrifuged at 50g for 2 minutes, and the supernatant was discarded. Then, the cells were resuspended in PBS. The average fluorescence intensity MFI of living cells was measured by flow cytometry, and GraphPad Prism5 software was used for nonlinear fitting and Kd value calculation. Data showed that ligand modified GalNAc-siRNAs promoted endocytosis/uptake by hepatocytes in vitro without the addition of transfection reagents, achieving delivery to hepatocytes. At the same time, GalNAc-siRNAs with different GalNAc structures exhibited certain differences in cell endocytosis and receptor binding abilities (Figure 6), wherein A1-A6 corresponded to E1164-GN1-E1164-GN6 respectively.

**Table 8 Different GalNAc modified siRNAs**

| Test group | Sequence (5'->3') |
|---|---|
| E1164-GN1 | Cy5-L96-AfsGmsAfCmCfUmGfUmUfUfUfGmCfUmUfUmUfGmUfAmAf(SEQ ID NO:35) |
| | UmsUfsAmCfAmAfAmAfGmCfAmAmAmAfCmAfGmGfUmCfUmsAmsGm(SEQ ID NO:8) |
| E1164-GN2 | Cy5-AfsGmsAfCmCfUmGfUmUfUfUfGmCfUmUfUmUfGmUfAmAf-L96(SEQ ID NO:36) |
| | UmsUfsAmCfAmAfAmAfGmCfAmAmAmAfCmAfGmGfUmCfUmsAmsGm(SEQ ID NO:8) |
| E1164-GN3 | Cy5-AfsGmsAfCmCfUmGfUmUfUfUfGmCfUmUfUmUfGmUfAmAf-XXX(SEQ ID NO:37) |
| | UmsUfsAmCfAmAfAmAfGmCfAmAmAmAfCmAfGmGfUmCfUmsAmsGm(SEQ ID NO:8) |
| E1164-GN4 | Cy5-X-AfsGmsAfCmCfUmGfUmUfUfUfGmCfUmUfUmUfGmUfAmAf-X(SEQ ID NO:38) |
| | UmsUfsAmCfAmAfAmAfGmCfAmAmAmAfCmAfGmGfUmCfUmsAmsGm(SEQ ID NO:8) |
| E1164-GN5 | Cy5-XXX-AfsGmsAfCmCfUmGfUmUfUfUfGmCfUmUfUmUfGmUfAmAf(SEQ ID NO:39) |
| | UmsUfsAmCfAmAfAmAfGmCfAmAmAmAfCmAfGmGfUmCfUmsAmsGm(SEQ ID NO:8) |
| E1164-GN6 | Cy5-AfsGmsAfCmCfUmGfUmUfUfUfGmCfUmUfUmUfGmUfAmAfGmCmAmGmCmCm dG-XXXX-dAGmGmCmUmsGmsCm(SEQ ID NO:40) |
| | UmsUfsAmCfAmAfAmAfGmCfAmAmAmAfCmAfGmGfUmCfUmsAmsGm(SEQ ID NO:8) |

In the above table, Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone, and Cy5 means Cyanine 5 fluorescent dye. The structure of L96 is shown in formula IV:

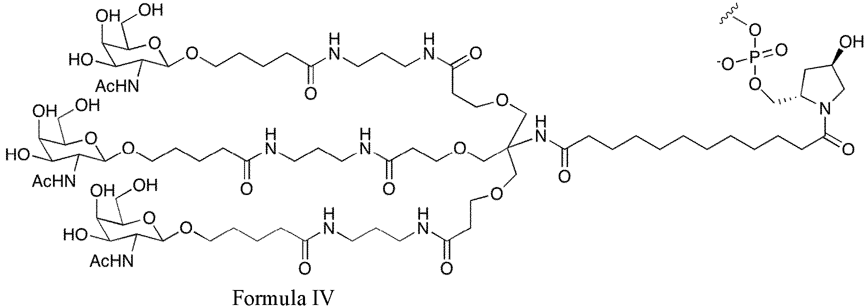

X has the structure of formula I: 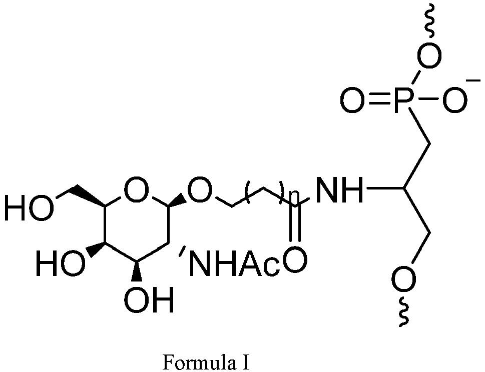 wherein n=3, two adjacent Xs in XX are linked by a phosphodiester bond or a phosphorothioate bond; X and XXX are both linked to the 5' end, 3' end or a middle nucleotide of the sense strand nucleotide sequence of the siRNA by a phosphodiester bond or a phosphorothioate bond.

The experiment used 36 male, 6- to 7-week-old SPF grade C57 mice (Shanghai model organisms Co., Ltd.), which were randomly divided into 6 groups, with 6 animals in each group, and administered by subcutaneous injection at the dosage of 5mg/kg (see Table 9 for experimental design). In vivo imaging was performed on all animals at 2h, 4h, 24h, and 48h after administration. The animals were euthanized 48 hours after administration, and the heart, spleen, lungs, liver, and kidneys were removed for organ imaging ex vitro (Figure 7).

**Table 9 Liver targeting experimental design**

| group | animal | Numbering of the mouse |
|---|---|---|
| E1164-GN1 | C57, male, six weeks old | 1#, 2#, 3#, 4#, 5#, 6# |
| E1164-GN2 | C57, male, six weeks old | 7#, 8#, 9#, 10#, 11#, 12# |
| E1164-GN3 | C57, male, six weeks old | 13#, 14#, 15#, 16#, 17#, 18# |
| E1164-GN4 | C57, male, six weeks old | 19#, 20#, 21#, 22#, 23#, 24# |
| E1164-GN5 | C57, male, six weeks old | 25#, 26#, 27#, 28#, 29#, 30# |
| E1164-GN6 | C57, male, six weeks old | 31#, 32#, 33#, 34#, 35#, 36# |

The results of in vivo imaging and ex vivo imaging analysis (Figure 7) showed that the order was that from left to right, top to bottom, corresponded to mice 1#-36# in turn. For example, the first row represented mice 1#-12#. 2 hours after administration, the fluorescence intensity of livers in different GalNAc-modified E1164 groups was concentrated in the liver. After 24 hours, most of the GalNAc sequences were metabolized, and the fluorescence intensity showed that different structures of GalNAc all had certain targeting properties to the liver.

### III. In vivo drug efficacy experiment of GalNAc structures

The experiment used 6-8 weeks old humanized PCSK9 male and female mice (Shanghai model organisms Co., Ltd.). A total of 50 mice were randomly divided into groups according to body weight, with 10 mice in each group (5 males and 5 males), and were subcutaneously injected with a single dose of 3 mg/kg (see Table 10 for administration groups). Blood was collected on days -7, -3, 4, 7, 11, 14, 18, 21, 25, 32, and 39 (fast for 6 hours before blood collection, and the first dose was day 0), and 0.1ml blood was collected by bleeding from the back of the eyeball. Serum was separated from the collected blood, and was used to detect the expression level of PCSK9 protein in serum with Human PCSK9 ELISA Kit (SinoBiological), which was compared between groups.

**Table 10 Experimental protocol for subcutaneous injection of siRNA drugs into mice**

| group | animal | Numbering of the mouse |
|---|---|---|
| NC | Humanized PCSK9, 5 males and 5 females, 6 weeks old | #1-#10 |
| E1164-GN1 | Humanized PCSK9, 5 males and 5 females, 6 weeks old | #11-#20 |
| E1164-GN2 | Humanized PCSK9, 5 males and 5 females, 6 weeks old | #21-#30 |
| E1164-GN3 | Humanized PCSK9, 5 males and 5 females, 6 weeks old | #31-#40 |
| E1164-GN4 | Humanized PCSK9, 5 males and 5 females, 6 weeks old | #41-#50 |

**Table 11 Average PCSK9 protein normalized to pre-treatment from Example 3.III**

| | Day 4 | Day 7 | Day 11 | Day 14 | Day 18 | Day 21 | Day 25 | Day 32 | Day 39 |
|---|---|---|---|---|---|---|---|---|---|
| NC | 1.00 | 1.00 | 1.00 | 1.00 | 1.00 | 1.00 | 1.00 | 1.00 | 1.00 |
| E1164-GN1 | 0.25 | 0.37 | 0.42 | 0.42 | 0.52 | 0.58 | 0.67 | 0.75 | 0.60 |
| E1164-GN2 | 0.30 | 0.34 | 0.48 | 0.49 | 0.50 | 0.66 | 0.67 | 0.70 | 0.67 |
| E1164-GN3 | 0.20 | 0.31 | 0.23 | 0.25 | 0.31 | 0.37 | 0.43 | 0.33 | 0.66 |
| E1164-GN4 | 0.25 | 0.53 | 0.50 | 0.71 | 0.70 | 0.67 | 0.68 | 0.67 | 0.73 |

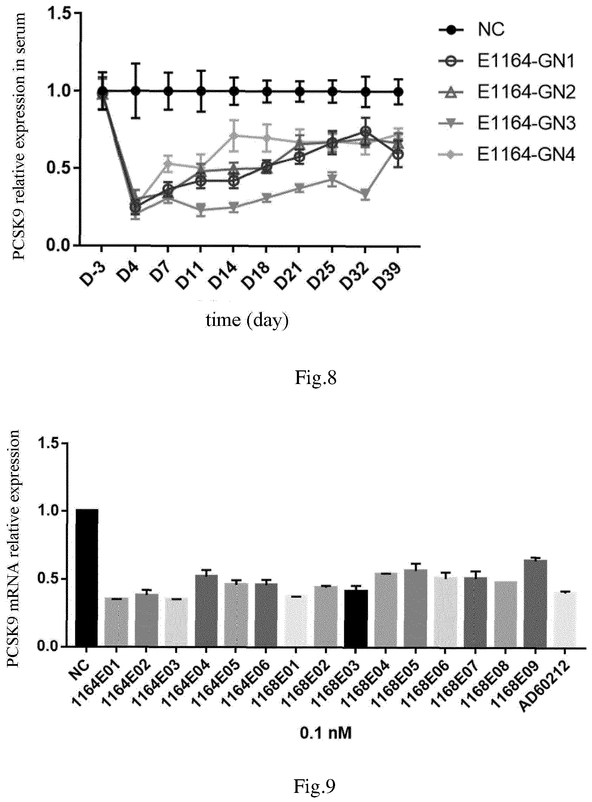

The experimental results showed (Figure 8, Table 11) that among E1164-GN1, E1164-GN2, E1164-GN3, and E1164-GN molecules, the PCSK9 protein decreased by up to 80% on day 4, and could still be reduced to about 60% on day 39; At the same time, the results showed that both the continuous series connection of three GalNAc structures and the coupled trident GalNAc structure could achieve the reduction and persistence of PCSK9 protein. The 3'-end coupled GalNAc structure was more durable and had a delayed rebound than the 5'-end drug effect.

### Example 4. Optimization of chemical modification platform

### I. Determination of inhibitory activity

We optimized the sequence modifications of U1164 and U1168 respectively (Table 12), and combined fluoro and methoxy modifications at different positions. The overall modification strategy used methoxy modifications to replace the antisense chain as far as possible. The steps of the synthesis of the sequences, the transfection of the Hep3B cell and the quantitative PCR detection and analysis were the same as those in Example 1, and the final concentration of transfection was 0.1 nM.

**Table 12 combinations of siRNA modification strategies for candidate sequences**

| name | Sequence (5'->3') | length | Molecular weight (g/mole) | GC content |
|---|---|---|---|---|
| 1164 E01 | AfsGmsAfCmCfUmGfUmUfUfUfGmCfUmUfUmUfGmU fAmAf-L96(SEQ ID NO:7) | 21 | 8583.94 | 33.3 |
| | UmsUfsAmCfAmAfAmAfGmCfAmAmAmAfCmAfGmGf UmCfUmsAfsGm(SEQ ID NO:41) | 23 | 7651.59 | 34.8 |
| 1164 E02 | AfsGmsAfCmCfUmGfUmUfUfUfGmCfUmUfUmUfGmU fAmAf-L96(SEQ ID NO:7) | 21 | 8583.94 | 33.3 |
| | UmsUfsAmCfAmAfAmAfGmCfAmAmAmAfCmAfGmGf UmCfUmsAmsGm(SEQ ID NO:8) | 23 | 7663.59 | 34.8 |
| 1164 E03 | AfsGmsAfCmCfUmGfUmUfUfUfGmCfUmUmUmUmGm UmAmAm-L96(SEQ ID NO:42) | 21 | 8601.94 | 33.3 |
| | UmsUfsAmCfAmAmAmAfGmCfAmAmAmAfCmAfGm GmUmCfUmsAmsGm(SEQ ID NO:43) | 23 | 7687.59 | 34.8 |
| 1164 E04 | AmsGmsAmCmCmUmGfUmUfUmUmGmCmUmUmUm UmGmUmAmAm-L96(SEQ ID NO:44) | 21 | 8703.94 | 33.3 |
| | UmsUfsAmCfAmAmAmAfGmCfAmAmAmAfCmAfGm GmUmCfUmsAmsGm(SEQ ID NO:43) | 23 | 7687.59 | 34.8 |
| 1164 E05 | AmsGmsAmCmCmUmGfUmUfUmUmGmCmUmUmUm UmGmUmAmAm-L96(SEQ ID NO:44) | 21 | 8703.94 | 33.3 |
| | UmsUfsAmCfAfAfAmAfGmCfAmAfAmAfCmAfGmGfU mCmUmsAmsGm(SEQ ID NO:45) | 23 | 7651.59 | 34.8 |
| 1164 E06 | AmsGmsAmCmCmUmGfUmUfUm(dT)GmCmUmUmUm UmGmUmAmAm-L96(SEQ ID NO:46) | 21 | 868797 | 33.3 |
| | UmsUfsAmCfAfAfAmAfGmCfAmAfAmAfCmAfGmGfU mCmUmsAmsGm(SEQ ID NO:45) | 23 | 7651.59 | 34.8 |
| 1168 E01 | CfsUmsGfUmUfUmUfGmCfUfUfUmUfGmUfAmAfCmU fUmGf-L96(SEQ ID NO:13) | 21 | 8537.86 | 33.3 |
| | CmsAfsAmGfUmUfAmCfAmAfAmAmGmCfAmAfAmAf CmAfGmsGfsUm(SEQ ID NO:47) | 23 | 7674.63 | 34.8 |
| 1168 E02 | CfsUmsGfUmUfUmUfGmCfUfUfUmUfGmUfAmAfCmU fUmGf-L96(SEQ ID NO:13) | 21 | 8537.86 | 33.3 |
| | CmsAfsAmGfUmUfAmCfAmAfAmAmGmCfAmAfAmAf CmAfGmsGnsUm(SEQ ID NO:14) | 23 | 7686.63 | 34.8 |
| 1168 E03 | CfsUmsGfUmUfUmUfGmCfUfUfUmUfGmUmAmAmCm UmUmGm-L96(SEQ ID NO:48) | 21 | 8555.86 | 33.3 |
| | CmsAfsAmGfUmUmAmCfAmAfAmAmGmCfAmAfAm AmCmAfGmsGmsUm(SEQ ID NO:49) | 23 | 7710.63 | 34.8 |
| 1168 E04 | CmsUmsGmUmUmUmUfGmCfUmUmUmUmGmUmAm AmCmUmUmGm-L96(SEQ ID NO:50) | 21 | 8657.86 | 33.3 |
| | CmsAfsAmGfUmUmAmCfAmAfAmAmGmCfAmAfAm AmCmAfGmsGmsUm(SEQ ID NO:49) | 23 | 7710.63 | 34.8 |
| 1168 E05 | CmsUmsGmUmUmUmUfGmCfUmUmUmUmGmUmAm AmCmUmUmGm-L96(SEQ ID NO:50) | 21 | 8657.86 | 33.3 |
| | CmsAfsAmGfUfUfAmCfAmAfAmAfGmCfAmAfAmAfC mAmGmsGmsUm(SEQ ID NO:51) | 23 | 7674.63 | 34.8 |
| 1168 E06 | CmsUmsGmUmUmUmUfGmCfUm(dT)UmUmGmUmAm AmCmUmUmGm-L96(SEQ ID NO:52) | 21 | 8641.89 | 33.3 |
| | CmsAfsAmGfUfUfAmCfAmAfAmAfGmCfAmAfAmAfC mAmGmsGmsUm(SEQ ID NO:51) | 23 | 7674.63 | 34.8 |
| 1168 E07 | CmsUmsGmUmUmUmUfGmCfUfUfUmUmGmUmAmA mCmUmUmGm-L96(SEQ ID NO:53) | 21 | 8634.4 | 33.3 |
| | CmsAfsAmGmUmUfAmCfAfAmAmAmGmCfAmAfAm AmCmAmGmsGmsUm(SEQ ID NO:54) | 23 | 7723.2 | 34.8 |
| 1168 E08 | CmsUmsGmUmUmUmUfGmCfUfUfUmUmGmUmAmA mCmUmUmGm-L96(SEQ ID NO:53) | 21 | 8634.4 | 33.3 |
| | CmsAfsAmGmUmUfAmCmAmAmAmAmGmCfAmAfA mAmCmAmGmsGmsUm(SEQ ID NO:55) | 23 | 7747.3 | 34.8 |
| 1168 E09 | CmsUmsGmUmUmUmUfGmCfUfUfUmUmGmUmAmA mCmUmUmGm-L96(SEQ ID NO:53) | 21 | 8634.4 | 33.3 |
| | CmsAfsAmGmUmUmAmCmAmAmAmAmGmCfAmAfA mAmCmAmGmsGmsUm(SEQ ID NO:56) | 23 | 7759.4 | 34.8 |
| AD60212 | CmsUmsAmGmAmCmCfUmGfUmTUmUmGmCmUmU mUmUmUmGmUm-L96(SEQ ID NO:245) | 21 | 8640.9 | 38.1 |
| | AmsCfsAmAfAfAfGmCfAmAfAmAfCmAfGmGfUmCfU mAmGmsAmsAm(SEQ ID NO:246) | 23 | 7697.67 | 34.8 |

In the above table, Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone, and the structure of L96 is shown in formula IV:

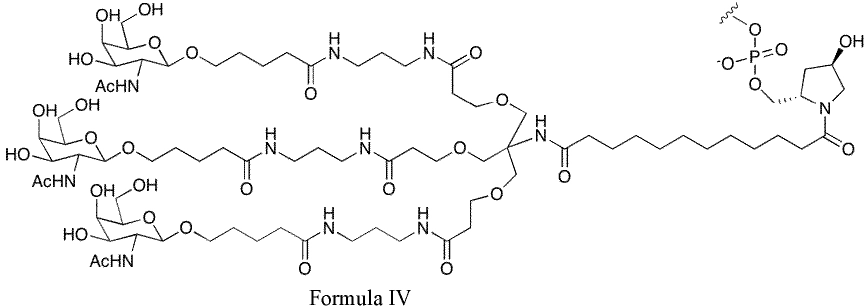

As shown in Figure 9, the scheme of modifications was important and certain modifications reduced inhibitory activity. The preferred modification scheme had high inhibitory activity: (1) when the antisense strand had an overhang of 5'(s)mN(s)mN3' structure at the 3' end, it facilitated the loading of the antisense strand into RISC and enhanced RNAi interference activity without affecting stability. (2) when the antisense strand was modified with fluoro at least at positions 2, 6, 14, and 16 from the 5' end, and other positions were modified with methoxy groups as far as possible, it facilitated RISC binding; (3) when the antisense strand was modified with at least two thio modifications starting from the 3' end and the 5' end, it was beneficial for maintaining the stability of the nucleic acid; (4) When the sense strand was modified with fluoro at position 7 and positions 9-11continuously from the 5' end, and other positions were subjected to methoxy modification as far as possible, it facilitated RISC binding; (5) The sense strand had at least two thio modifications starting from the 5' end, and the 3' end was covalently coupled with GalNAc. Next, sequence U1168 was selected for screening with different chemical modifications.

### II. In vivo efficacy experiments of different chemically modified sequence 1168

The experiment used 6-8 weeks old humanized PCSK9 male and female mice (Shanghai model organisms Co., Ltd.), a total of 25, which were randomly divided into groups according to body weight, with 5 mice in each group, and were administered with a single subcutaneous injection of 3 mg/kg (The administration groups are shown in Table 12). Blood was collected on days -7, -3, 4, 7, 11, 14, 18, 21 and 25 (fast for 6 hours before blood collection, and the first dose was day 0), and 0.1ml blood was collected by bleeding from the back of the eyeball. Serum was separated from the collected blood, and was used to detect the expression level of PCSK9 protein in serum with Human PCSK9 ELISA Kit (SinoBiological), which was compared between groups.

**Table 13 Experimental protocol for subcutaneous injection of siRNA drugs into mice**

| group | animal | Numbering of the mouse |
|---|---|---|
| NC | Humanized PCSK9, 5 males and females in total, six weeks old | #1-#5 |
| E1168 | Humanized PCSK9, 5 males and females in total, six weeks old | #6-#10 |
| 1168 E07 | Humanized PCSK9, 5 males and females in total, six weeks old | #11-#15 |
| 1168 E08 | Humanized PCSK9, 5 males and females in total, six weeks old | #16-#20 |
| 1168 E09 | Humanized PCSK9, 5 males and females in total, six weeks old | #21-#25 |

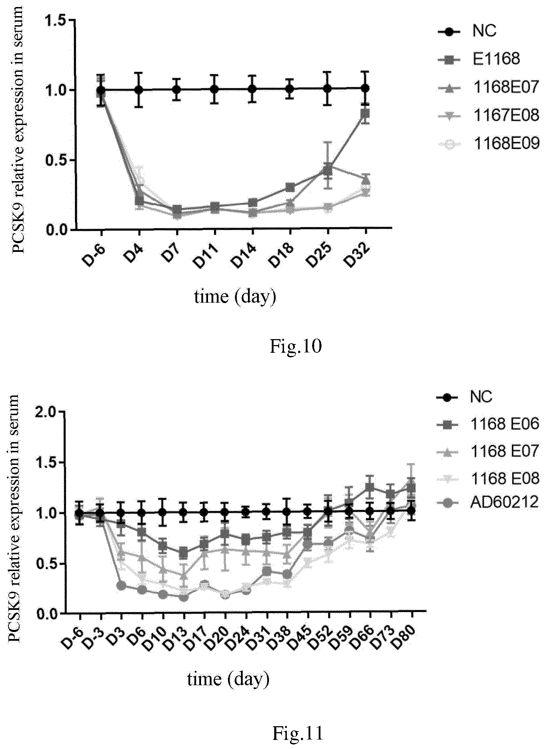

The experimental results showed (Figure 10) that sequence 1168 with different chemical modifications reached the lowest point on day 11, and the PCSK9 protein reduced by nearly 90%. As the number of fluorinated bases decreased, the rebound of the PCSK9 protein slowed down, and the persistence increased. The efficacy of sequences 1168E08 and E09 was long-lasting.

### Example 5. Test of in vivo effectiveness in mice

The experiment used 6-8 weeks old humanized PCSK9 male and female mice (Shanghai model organisms Co., Ltd.), a total of 50, which were randomly divided into groups according to body weight, with 10 mice in each group (5 males and 5 males), and were administered with a single subcutaneous injection of 3 mg/kg (The administration groups are shown in Table 13). Blood was collected on days -6, -3, 3, 6, 10, 13, 17, 20, 24, 31, 38, 45, 52, 59, 66, 73 and 80 (fast for 6 hours before blood collection, and the first dose was day 0), and 0.1ml blood was collected by bleeding from the back of the eyeball. Serum was separated from the collected blood, and was used to detect the expression level of PCSK9 protein in serum with Human PCSK9 ELISA Kit (SinoBiological), which was compared between groups.

**Table 14 Experimental protocol for subcutaneous injection of siRNA drugs into mice**

| group | animal | Numbering of the mouse |
|---|---|---|
| NC | Humanized PCSK9, 5 males and 5 females, six weeks old | #1-#10 |
| 1168 E06 | Humanized PCSK9, 5 males and 5 females, six weeks old | #11-#20 |
| 1168 E07 | Humanized PCSK9, 5 males and 5 females, six weeks old | #21-#30 |
| 1168 E08 | Humanized PCSK9, 5 males and 5 females, six weeks old | #31-#40 |
| AD60212 | Humanized PCSK9, 5 males and 5 females, six weeks old | #41-#50 |

**Table 15 Average PCSK9 protein normalized to pre-treatment from Example 5**

| | NC | 1168E06 | 1168E07 | 1168E08 | AD60212 |
|---|---|---|---|---|---|
| day 3 | 1.00 | 0.89 | 0.62 | 0.52 | 0.28 |
| day 6 | 1.00 | 0.81 | 0.56 | 0.34 | 0.24 |
| day 10 | 1.00 | 0.67 | 0.44 | 0.29 | 0.19 |
| day 13 | 1.00 | 0.60 | 0.38 | 0.22 | 0.16 |
| day 17 | 1.00 | 0.69 | 0.60 | 0.26 | 0.28 |
| day 20 | 1.00 | 0.78 | 0.63 | 0.18 | 0.18 |
| day 24 | 1.00 | 0.73 | 0.61 | 0.26 | 0.23 |
| day 31 | 1.00 | 0.75 | 0.60 | 0.31 | 0.41 |
| day 38 | 1.00 | 0.80 | 0.58 | 0.29 | 0.38 |
| day 45 | 1.00 | 0.79 | 0.80 | 0.49 | 0.68 |
| day 52 | 1.00 | 1.01 | 0.98 | 0.57 | 0.68 |
| day 59 | 1.00 | 1.08 | 1.01 | 0.71 | 0.81 |
| day 66 | 1.00 | 1.23 | 0.79 | 0.68 | 0.73 |
| day 73 | 1.00 | 1.17 | 1.09 | 0.79 | 1.01 |
| day 80 | 1.00 | 1.22 | 1.31 | 1.08 | 1.05 |

The efficacy results in mice showed (Figure 11, Table 15) that the PCSK9 protein level of the positive control AD60212 decreased by 80% on day 10, until rebounded after day 31, and returned to the pre-administration level on day 73. Among the candidate molecules, 1168E06 and 1168E07 were less effective than the positive control, and returned to the pre-administration level on day 59. The 1168E08 molecule reached the lowest point on day 13, with a decrease of 80%, and then rebounded until day 38, and returned to the pre-administration level on day 80, which had a rebound period one week behind that of the positive control.

### Example 6. Test of in vivo effectiveness in cynomolgus monkeys

Eight cynomolgus monkeys (Beijing Joinn Biologies Co., Ltd.) for experiment use were randomly assigned to the positive control group and the experimental test group (AD60212, 1168E06, 1168E07, 1168E08, four test compounds) according to body weight. There were a total of 4 groups, each group had 2 animals, one female and one male. The animals were administered with a single subcutaneous injection of 5 mg/kg drug, and blood was collected from the cephalic vein of the forelimbs on days -1, 2, 5, 8, 11, 15, 18, 22, 25, 29, 32, 36, 43, 50, 57, 64, 71, 78, 85, 92, 99, 106, 113, and 120 (fast before blood collection, and the first dose was day 0). Serum was separated for PCSK9 protein and LDL-C (low-density lipoprotein cholesterol) detection. After day 120, PCSK9 protein and LDL-C were continued to be detected until they returned to pre-administration levels.

### I. LDL-C detection

### 1. Method

The concentration of LDL-C in serum was detected using Toshiba TBA120 blood biochemical analyzer and was compared between groups.

### 2. Result

**Table 16 Average LDL-c levels normalized to pre-treatment from Example 6**

| | 1168E06 | 1168E07 | 1168E08 | AD60212 |
|---|---|---|---|---|
| day 2 | 1.09 | 1.11 | 0.80 | 1.02 |
| day 5 | 0.75 | 0.81 | 0.67 | 0.71 |
| day 8 | 0.90 | 0.76 | 0.71 | 0.70 |
| day 11 | 0.77 | 0.48 | 0.35 | 0.43 |
| day 15 | 0.74 | 0.42 | 0.37 | 0.51 |
| day 18 | 0.69 | 0.39 | 0.30 | 0.38 |
| day 22 | 0.72 | 0.40 | 0.36 | 0.49 |
| day 25 | 0.65 | 0.40 | 0.27 | 0.33 |
| day 29 | 1.00 | 0.34 | 0.34 | 0.39 |
| day 32 | 0.92 | 0.38 | 0.33 | 0.34 |
| day 36 | 0.77 | 0.44 | 0.29 | 0.42 |
| day 43 | 1.07 | 0.40 | 0.37 | 0.48 |
| day 50 | 0.83 | 0.46 | 0.34 | 0.52 |
| day 57 | 0.83 | 0.43 | 0.39 | 0.50 |
| day 64 | 0.71 | 0.37 | 0.32 | 0.44 |
| day 71 | 0.91 | 0.44 | 0.36 | 0.54 |
| day 78 | 0.82 | 0.46 | 0.39 | 0.53 |
| day 85 | 0.88 | 0.58 | 0.39 | 0.65 |
| day 92 | 0.88 | 0.53 | 0.38 | 0.61 |
| day 99 | 0.79 | 0.56 | 0.46 | 0.64 |
| day 106 | 0.85 | 0.64 | 0.36 | 0.59 |
| day 113 | 0.83 | 0.52 | 0.43 | 0.71 |
| day 120 | 0.92 | 0.69 | 0.60 | 0.80 |

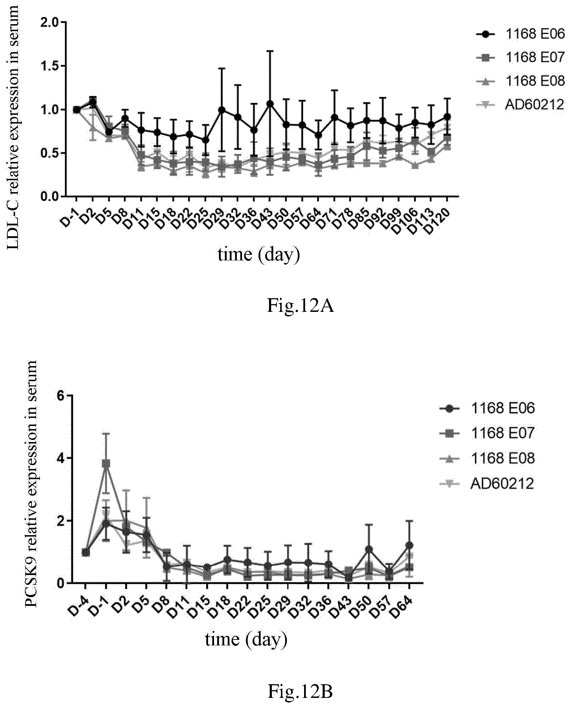

Experimental results (Figure 12A, Table 16) showed that compared with before administration, the serum LDL-C levels of the 1168E06, 1168E07, 1168E08, and AD60212 groups decreased by 23%, 52%, 65%, and 57% respectively on day 11 after administration. Subsequently, the effects of 1168E07 and 1168E08 molecules in reducing LDL-C were more significant than that of the positive control, and continued until day 100 when the LDL-C rebound began.

### 2. PCSK9 protein detection

### 1. Method

The expression level of PCSK9 protein in serum was detected using Monkey Proprotein convertase subtilisin/kexin type 9 (PCSK9) ELISA kit (CUSABIO) and was compared between groups.

### 2. Result

**Table 17 Average PCSK9 protein normalized to pre-treatment from Example 6**

| | 1168E06 | 1168E07 | 1168E08 | AD60212 |
|---|---|---|---|---|
| day 2 | 0.83 | 0.50 | 0.95 | 0.56 |
| day 5 | 0.79 | 0.37 | 0.82 | 0.63 |
| day 8 | 0.23 | 0.28 | 0.26 | 0.28 |
| day 11 | 0.50 | 0.15 | 0.21 | 0.31 |
| day 15 | 0.29 | 0.08 | 0.11 | 0.17 |
| day 18 | 0.36 | 0.13 | 0.24 | 0.24 |
| day 22 | 0.31 | 0.08 | 0.16 | 0.17 |
| day 25 | 0.25 | 0.09 | 0.19 | 0.18 |
| day 29 | 0.30 | 0.09 | 0.12 | 0.17 |
| day 32 | 0.28 | 0.08 | 0.13 | 0.16 |
| day 36 | 0.28 | 0.09 | 0.14 | 0.19 |
| day 43 | 0.10 | 0.11 | 0.07 | 0.12 |
| day 50 | 0.50 | 0.15 | 0.13 | 0.24 |
| day 57 | 0.18 | 0.06 | 0.12 | 0.15 |
| day 64 | 0.57 | 0.14 | 0.25 | 0.40 |

The results (Figure 12B, Table 17) showed that compared with before administration, the serum PCSK9 protein levels of the 1168E06, 1168E07, 1168E08, and AD60212 groups dropped to the lowest point on day 15 after administration, reducing by 71%, 92%, 89%, and 83% respectively, and remained low. Subsequently, the effects of 1168E07 and 1168E08 molecules on reducing PCSK9 protein were more significant than that of the positive control, and continued until day 57 when the rebound began.

### 3. Pharmacokinetic test

Serum collected from cynomolgus monkeys at different times was quantitatively analyzed by mass spectrometry, and the drug concentration levels at different times were obtained by calibration with the standard curve of the standard substance.

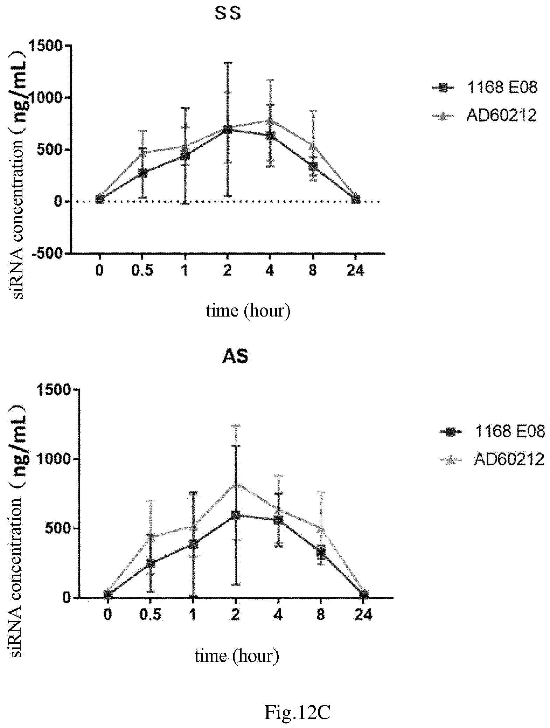

The experimental results (Figure 12C) showed that the pharmacokinetic curves of the sense and antisense strands of the positive control AD60212 and 1168E08 sequences were similar, reaching the peak at 2h, and the siRNA concentration was undetectable at 24h.

### Example 7. Toxicological studies on mice

A total of 21 male, 6 to 7 week old SPF grade C57 mice (Shanghai model organisms Co., Ltd.) were used in the experiment. They were randomly assigned according to body weight into a blank control group, a positive control group, and an experimental test group (NC, AD60212, 1168E08), with a total of 3 groups, 7 animals in each group, and were administered with a single subcutaneous injection of 400mg/kg. Blood was collected at 72, 96, and 120 hours (fasting for 6 hours before blood collection, and the first dose was on day 0), and 0.1 ml of blood was collected by bleeding from the back of the eyeball. The collected blood was used to detect the changes of the concentrations of ALT and AST in the serum using a Toshiba TBA120 blood biochemical instrument, and comparisons were made between groups.

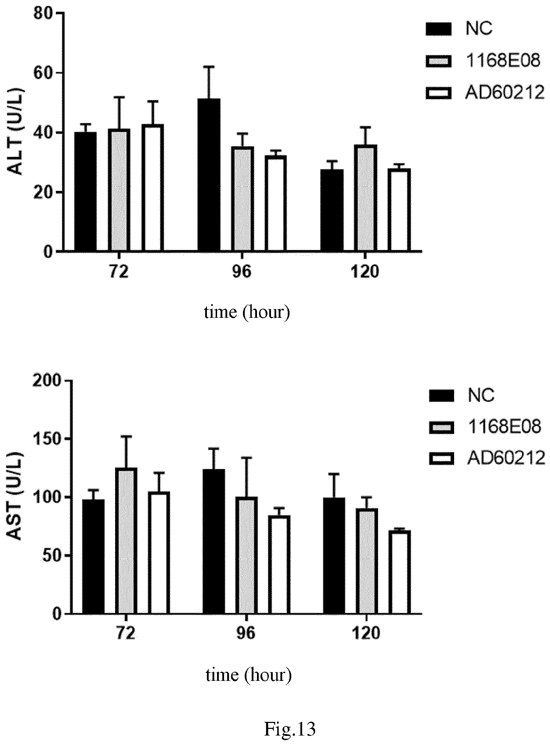

The results of mouse experiments showed (Figure 13) that the positive control AD60212 and the candidate molecule 1168E08 performed well in terms of the changes of ALT and AST in serum at a dose of 400mpk without obvious toxicity.

## Claims

1. A double-stranded RNAi agent that can inhibit the expression of PCSK9 in a cell, wherein the double-stranded RNAi agent comprises a sense strand and an antisense strand, wherein the sense strand is complementary to the antisense strand, and the antisense strand comprises a sequence complementary to a portion of the mRNA encoding PCSK9, wherein each strand is 14 to 30 nucleotides in length, and the sense strand nucleotide sequence in the double-stranded RNAi agent is selected from 14 to 30 nucleotides in SEQ ID NO:1 or SEQ ID NO:2.

2. The double-stranded RNAi agent according to claim 1, **characterized in that** the sense strand and the antisense strand have 17-30 nucleotides, preferably 17-25 nucleotides, and more preferably 19-23 nucleotides.

3. The double-stranded RNAi agent according to claim 2, **characterized in that** the sense strand has 21 nucleotides and the antisense strand has 23 nucleotides.

4. The double-stranded RNAi agent according to any one of claims 1 to 3, **characterized in that** one or more nucleotides in the sense and antisense strands have one or more modifications selected from the group consisting of: 2'-methoxyethyl , 2'-O-alkyl, 2'-O-allyl, 2'-C-allyl, 2'-fluoro, 2'-deoxy, 2'-hydroxy, locked nucleic acid modification, ring-opening or non-locked nucleic acid modification, DNA modification, fluorescent probe modification.

5. The double-stranded RNAi agent according to claim 4, **characterized in that** the modification is 2'-O-methyl and/or 2'-fluoro modification.

6. The double-stranded RNAi agent according to any one of claims 1 to 5, **characterized in that** the double-stranded RNAi agent further comprises at least one phosphorothioate or methylphosphonate internucleotide bond, preferably at least one phosphorothioate bond.

7. The double-stranded RNAi according to claim 6, **characterized in that** the phosphorothioate or methylphosphonate internucleotide bond is at the 5' and 3' ends of one strand, preferably, the inter-nucleotide bond is between 3 nucleotides at the 5' and 3' ends of the sense strand and the antisense strand.

8. The double-stranded RNAi agent according to any one of claims 4 to 7, **characterized in that** the double-stranded RNAi agent comprises: (1) the antisense strand has an overhang of 5'(s)mN(s)mN3' structure at the 3' end; (2) the antisense strand is modified with fluoro at least at positions 2, 6, 14, and 16 from the 5' end, and is modified with methoxy at other positions; (3) the antisense strand is modified with at least two thio groups starting from the 3' end and the 5' end; (4) the sense strand is modified with fluoro at position 7 and positions 9-11continuously from the 5' end, and other positions are modified with methoxy; (5) the sense strand has at least two thio modifications starting from the 5' end.

9. The double-stranded RNAi agent according to any one of claims 1 to 8, **characterized in that** the sense strand is conjugated to at least one ligand selected from the group consisting of cholesterol, biotin, vitamins, galactose derivatives or analogs, lactose derivatives or analogs, N-acetylgalactosamine derivatives or analogs, N-acetylglucosamine derivatives or analogs; preferably, the ligand is linked to the 3' end of the sense strand.

10. The double-stranded RNAi agent according to claim 9, **characterized in that** the ligand is one or more GalNAc derivatives linked to a monovalent or trivalent branched linker.

11. The double-stranded RNAi agent according to any one of claims 1 to 10, **characterized in that** the double-stranded RNAi agent comprises:
(1) an antisense strand consisting of a nucleotide sequence having at least 90%, preferably 95%, 96%, 97%, 98%, 99% or 100% identity with nucleotide sequence UfsGmsUfCmCfUmCfUmCfUfGfUmUfGmCfCmUfUmUfUmUf and a sense strand consisting of a nucleotide sequence having at least 90%, preferably 95%, 96%, 97%, 98%, 99% or 100% identity with nucleotide sequence AmsAfsAmAfAmGfGmCfAmAfCmAmGmAfGmAfGmGfAmCfAmsGmsAm;
(2) an antisense strand consisting of a nucleotide sequence having at least 90%, preferably 95%, 96%, 97%, 98%, 99% or 100% identity with nucleotide sequence GfsUmsCfCmUfCmUfCmUfGfUfUmGfCmCfUmUfUmUfUmAf and a sense strand consisting of a nucleotide sequence having at least 90%, preferably 95%, 96%, 97%, 98%, 99% or 100% identity with nucleotide sequence UmsAfsAmAfAmAfGmGfCmAfAmCmAmGfAmGfAmGfGmAfCmsAmsGm;
(3) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(4) an antisense strand consisting of a nucleotide sequence having at least 90%, preferably 95%, 96%, 97%, 98%, 99% or 100% identity with nucleotide sequence GfsAmsCfCmUfGmUfUmUfUfGfCmUfUmUfUmGfUmAfAmCf and a sense strand consisting of a nucleotide sequence having at least 90%, preferably 95%, 96%, 97%, 98%, 99% or 100% identity with nucleotide sequence GmsUfsUmAfCmAfAmAfAmGfCmAmAmAfAmCfAmGfGmUfCmsUmsAm;
(5) an antisense strand consisting of a nucleotide sequence having at least 90%, preferably 95%, 96%, 97%, 98%, 99% or 100% identity with nucleotide sequence AfsCmsCfUmGfUmUfUmUfGfCfUmUfUmUfGmUfAmAfCmUf and a sense strand consisting of a nucleotide sequence having at least 90%, preferably 95%, 96%, 97%, 98%, 99% or 100% identity with nucleotide sequence AmsGfsUmUfAmCfAmAfAmAfGmCmAmAfAmAfCmAfGmGfUmsCmsUm;
(6) an antisense strand consisting of a nucleotide sequence having at least 90%, preferably 95%, 96%, 97%, 98%, 99% or 100% identity with nucleotide sequence CfsUmsGfUmUfUmUfGmCfUfUfUmUfGmUfAmAfCmUfUmGf and a sense strand consisting of a nucleotide sequence having at least 90%, preferably 95%, 96%, 97%, 98%, 99% or 100% identity with nucleotide sequence CmsAfsAmGfUmUfAmCfAmAfAmAmGmCfAmAfAmAfCmAfGmsGmsUm;
(7) an antisense strand consisting of a nucleotide sequence having at least 90%, preferably 95%, 96%, 97%, 98%, 99% or 100% identity with nucleotide sequence UfsUmsUfGmUfAmAfCmUfUfGfAmAfGmAfUmAfUmUfUmAf and a sense strand consisting of a nucleotide sequence having at least 90%, preferably 95%, 96%, 97%, 98%, 99% or 100% identity with nucleotide sequence UmsAfsAmAfUmAfUmCfUmUfCmAmAmGfUmUfAmCfAmAfAmsAmsGm;
(8) an antisense strand consisting of a nucleotide sequence having at least 90%, preferably 95%, 96%, 97%, 98%, 99% or 100% identity with nucleotide sequence UfsUmsGfUmAfAmCfUmUfGfAfAmGfAmUfAmUfUmUfAmUf and a sense strand consisting of a nucleotide sequence having at least 90%, preferably 95%, 96%, 97%, 98%, 99% or 100% identity with nucleotide sequence AmsUfsAmAfAmUfAmUfCmUfUmCmAmAfGmUfUmAfCmAfAmsAmsAm;
(9) an antisense strand consisting of a nucleotide sequence having at least 90%, preferably 95%, 96%, 97%, 98%, 99% or 100% identity with nucleotide sequence UfsGmsUfAmAfCmUfUmGfAfAfGmAfUmAfUmUfUmAfUmUf and a sense strand consisting of a nucleotide sequence having at least 90%, preferably 95%, 96%, 97%, 98%, 99% or 100% identity with nucleotide sequence AmsAfsUmAfAmAfUmAfUmCfUmUmCmAfAmGfUmUfAmCfAmsAmsAm;
(10) an antisense strand consisting of a nucleotide sequence having at least 90%, preferably 95%, 96%, 97%, 98%, 99% or 100% identity with nucleotide sequence GfsUmsAfAmCfUmUfGmAfAfGfAmUfAmUfUmUfAmUfUmCf and a sense strand consisting of a nucleotide sequence having at least 90%, preferably 95%, 96%, 97%, 98%, 99% or 100% identity with nucleotide sequence GmsAfsAmUfAmAfAmUfAmUfCmUmUmCfAmAfGmUfUmAfCmsAmsAm;
(11) an antisense strand consisting of a nucleotide sequence having at least 90%, preferably 95%, 96%, 97%, 98%, 99% or 100% identity with nucleotide sequence AfsUmsAfUmUfUmAfUmUfCfUfGmGfGmUfUmUfUmGfUmAf and a sense strand consisting of a nucleotide sequence having at least 90%, preferably 95%, 96%, 97%, 98%, 99% or 100% identity with nucleotide sequence UmsAfsCmAfAmAfAmCfCmCfAmGmAmAfUmAfAmAfUmAfUmsCmsUm;
(12) an antisense strand consisting of a nucleotide sequence having at least 90%, preferably 95%, 96%, 97%, 98%, 99% or 100% identity with nucleotide sequence UfsUmsUfAmUfUmCfUmGfGfGfUmUfUmUfGmUfAmGfCmAf and a sense strand consisting of a nucleotide sequence having at least 90%, preferably 95%, 96%, 97%, 98%, 99% or 100% identity with nucleotide sequence UmsGfsCmUfAmCfAmAfAmAfCmCmCmAfGmAfAmUfAmAfAmsUmsAm;
(13) an antisense strand consisting of a nucleotide sequence having at least 90%, preferably 95%, 96%, 97%, 98%, 99% or 100% identity with nucleotide sequence AfsUmsUfCmUfGmGfGmUfUfUfUmGfUmAfGmCfAmUfUmUf and a sense strand consisting of a nucleotide sequence having at least 90%, preferably 95%, 96%, 97%, 98%, 99% or 100% identity with nucleotide sequence AmsAfsAmUfGmCfUmAfCmAfAmAmAmCfCmCfAmGfAmAfUmsAmsAm;
(14) an antisense strand consisting of a nucleotide sequence having at least 90%, preferably 95%, 96%, 97%, 98%, 99% or 100% identity with nucleotide sequence CfsUmsGfGmGfUmUfUmUfGfUfAmGfCmAfUmUfUmUfUmAf and a sense strand consisting of a nucleotide sequence having at least 90%, preferably 95%, 96%, 97%, 98%, 99% or 100% identity with nucleotide sequence Um s Afs Am AfAm AfUm GfCmUfAm Cm Am AfAm AfCm CfCm AfGm s Am s Am;
(15) an antisense strand consisting of a nucleotide sequence having at least 90%, preferably 95%, 96%, 97%, 98%, 99% or 100% identity with nucleotide sequence UfsGmsGfGmUfUmUfUmGfUfAfGmCfAmUfUmUfUmUfAmUf and a sense strand consisting of a nucleotide sequence having at least 90%, preferably 95%, 96%, 97%, 98%, 99% or 100% identity with nucleotide sequence AmsUfsAmAfAmAfAmUfGmCfUmAmCmAfAmAfAmCfCmCfAmsGmsAm;
(16) an antisense strand consisting of a nucleotide sequence having at least 90%, preferably 95%, 96%, 97%, 98%, 99% or 100% identity with nucleotide sequence GfsGmsGfCmUfGmAfGmCfUfUfUmAfAmAfAmUfGmGfUmUf and a sense strand consisting of a nucleotide sequence having at least 90%, preferably 95%, 96%, 97%, 98%, 99% or 100% identity with nucleotide sequence Am s Afs Cm CfAmUfUmUfUm AfAm Am Gm CfUm CfAm GfCm CfCm s Cm s Am;
(17) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(18) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(19) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(20) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(21) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence Um sUfs Am CfAm AfAm AfGm CfAm Am Am AfCm AfGm GfUm CfUm s Am s Gm;
(22) an antisense strand consisting of a nucleotide sequence having at least 90%, preferably 95%, 96%, 97%, 98%, 99% or 100% identity with nucleotide sequence
AfsGmsAfCmCfUmGfUmUfUfUfGmCfUmUfUmUfGmUfAmAfGmCmAmGmCmCmdGd AGmGmCmUmsGmsCm and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAmsGm;
(23) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUfUmUfGmUfAmAf and a sense strand consisting of a nucleotide sequence having at least 90%, preferably 95%, 96%, 97%, 98%, 99% or 100% identity with nucleotide sequence UmsUfsAmCfAmAfAmAfGmCfAmAmAmAfCmAfGmGfUmCfUmsAfsGm;
(24) an antisense strand consisting of a nucleotide sequence having at least 90%, preferably 95%, 96%, 97%, 98%, 99% or 100% identity with nucleotide sequence AfsGmsAfCmCfUmGfUmUfUfUfGmCfUmUmUmUmGmUmAmAm and a sense strand consisting of a nucleotide sequence having at least 90%, preferably 95%, 96%, 97%, 98%, 99% or 100% identity with nucleotide sequence UmsUfsAmCfAmAmAmAfGmCfAmAmAmAfCmAfGmGmUmCfUmsAmsGm;
(25) an antisense strand consisting of a nucleotide sequence having at least 90%, preferably 95%, 96%, 97%, 98%, 99% or 100% identity with nucleotide sequence AmsGmsAmCmCmUmGfUmUfUmUmGmCmUmUmUmUmGmUmAmAm and a sense strand consisting of a nucleotide sequence having at least 90%, preferably 95%, 96%, 97%, 98%, 99% or 100% identity with nucleotide sequence UmsUfsAmCfAmAmAmAfGmCfAmAmAmAfCmAfGmGmUmCfUmsAmsGm;
(26) an antisense strand consisting of a nucleotide sequence having at least 90%, preferably 95%, 96%, 97%, 98%, 99% or 100% identity with nucleotide sequence AmsGmsAmCmCmUmGfUmUfUmUmGmCmUmUmUmUmGmUmAmAm and a sense strand consisting of a nucleotide sequence having at least 90%, preferably 95%, 96%, 97%, 98%, 99% or 100% identity with nucleotide sequence UmsUfsAmCfAfAfAmAfGmCfAmAfAmAfCmAfGmGfUmCmUmsAmsGm;
(27) an antisense strand consisting of a nucleotide sequence having at least 90%, preferably 95%, 96%, 97%, 98%, 99% or 100% identity with nucleotide sequence AmsGmsAmCmCmUmGfUmUfUm(dT)GmCmUmUmUmUmGmUmAmAm and a sense strand consisting of a nucleotide sequence having at least 90%, preferably 95%, 96%, 97%, 98%, 99% or 100% identity with nucleotide sequence UmsUfsAmCfAfAfAmAfGmCfAmAfAmAfCmAfGmGfUmCmUmsAmsGm;
(28) an antisense strand consisting of a nucleotide sequence having at least 90%, preferably 95%, 96%, 97%, 98%, 99% or 100% identity with nucleotide sequence CfsUmsGfUmUfUmUfGmCfUfUfUmUfGmUfAmAfCmUfUmGf and a sense strand consisting of a nucleotide sequence having at least 90%, preferably 95%, 96%, 97%, 98%, 99% or 100% identity with nucleotide sequence CmsAfsAmGfUmUfAmCfAmAfAmAmGmCfAmAfAmAfCmAfGmsGfsUm;
(29) an antisense strand consisting of a nucleotide sequence having at least 90%, preferably 95%, 96%, 97%, 98%, 99% or 100% identity with nucleotide sequence CfsUmsGfUmUfUmUfGmCfUfUfUmUfGmUmAmAmCmUmUmGm and a sense strand consisting of a nucleotide sequence having at least 90%, preferably 95%, 96%, 97%, 98%, 99% or 100% identity with nucleotide sequence CmsAfsAmGfUmUmAmCfAmAfAmAmGmCfAmAfAmAmCmAfGmsGmsUm;
(30) an antisense strand consisting of a nucleotide sequence having at least 90%, preferably 95%, 96%, 97%, 98%, 99% or 100% identity with nucleotide sequence CmsUmsGmUmUmUmUfGmCfUmUmUmUmGmUmAmAmCmUmUmGm and a sense strand consisting of a nucleotide sequence having at least 90%, preferably 95%, 96%, 97%, 98%, 99% or 100% identity with nucleotide sequence CmsAfsAmGfUmUmAmCfAmAfAmAmGmCfAmAfAmAmCmAfGmsGmsUm;
(31) an antisense strand consisting of a nucleotide sequence having at least 90%, preferably 95%, 96%, 97%, 98%, 99% or 100% identity with nucleotide sequence CmsUmsGmUmUmUmUfGmCfUmUmUmUmGmUmAmAmCmUmUmGm and a sense strand consisting of a nucleotide sequence having at least 90%, preferably 95%, 96%, 97%, 98%, 99% or 100% identity with nucleotide sequence Cm s Afs Am GfUfUfAm CfAm AfAm AfGm CfAm AfAm AfCm Am Gm s Gm sUm;
(32) an antisense strand consisting of a nucleotide sequence having at least 90%, preferably 95%, 96%, 97%, 98%, 99% or 100% identity with nucleotide sequence CmsUmsGmUmUmUmUfGmCfUm(dT)UmUmGmUmAmAmCmUmUmGm and a sense strand consisting of a nucleotide sequence having at least 90%, preferably 95%, 96%, 97%, 98%, 99% or 100% identity with nucleotide sequence Cm s Afs Am GfUfUfAm CfAm AfAm AfGm CfAm AfAm AfCm Am Gm s Gm sUm;
(33) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and a sense strand consisting of a nucleotide sequence having at least 90%, preferably 95%, 96%, 97%, 98%, 99% or 100% identity with nucleotide sequence Cm s Afs Am GmUmUfAm CfAfAm Am Am Gm CfAm AfAm Am Cm Am Gm s Gm sUm;
(34) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and a sense strand consisting of a nucleotide sequence having at least 90%, preferably 95%, 96%, 97%, 98%, 99% or 100% identity with nucleotide sequence CmsAfsAmGmUmUfAmCmAmAmAmAmGmCfAmAfAmAmCmAmGmsGmsUm; or (35) an antisense strand consisting of a nucleotide sequence having at least 90%, preferably 95%, 96%, 97%, 98%, 99% or 100% identity with nucleotide sequence CmsUmsGmUmUmUmUfGmCfUfUfUmUmGmUmAmAmCmUmUmGm and a sense strand consisting of a nucleotide sequence having at least 90%, preferably 95%, 96%, 97%, 98%, 99% or 100% identity with nucleotide sequence CmsAfsAmGmUmUmAmCmAmAmAmAmGmCfAmAfAmAmCmAmGmsGmsUm;
wherein Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone.

12. The double-stranded RNAi agent according to any one of claims 9-10, **characterized in that** the ligand structure is shown in formula II:

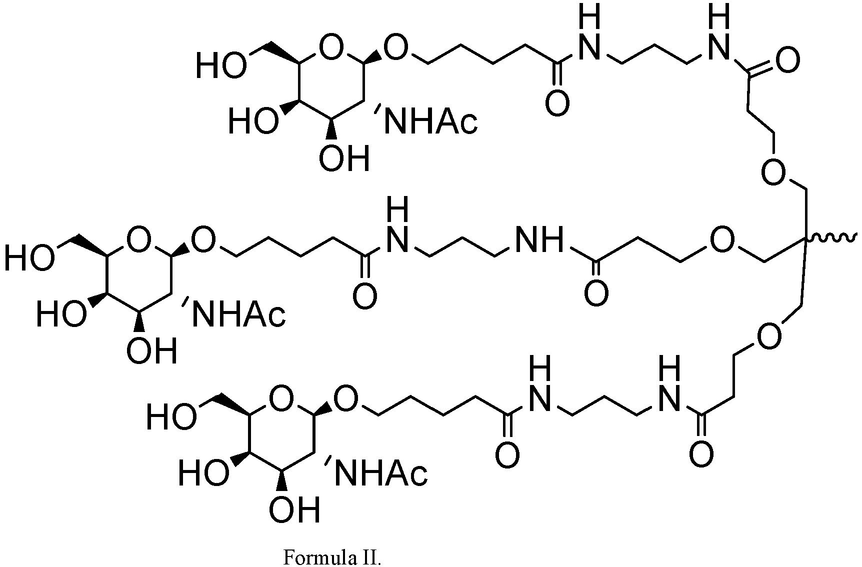

13. The double-stranded RNAi agent of any one of claims 9-12, wherein the double-stranded RNAi agent has the structure of formula III, wherein X is O or S:

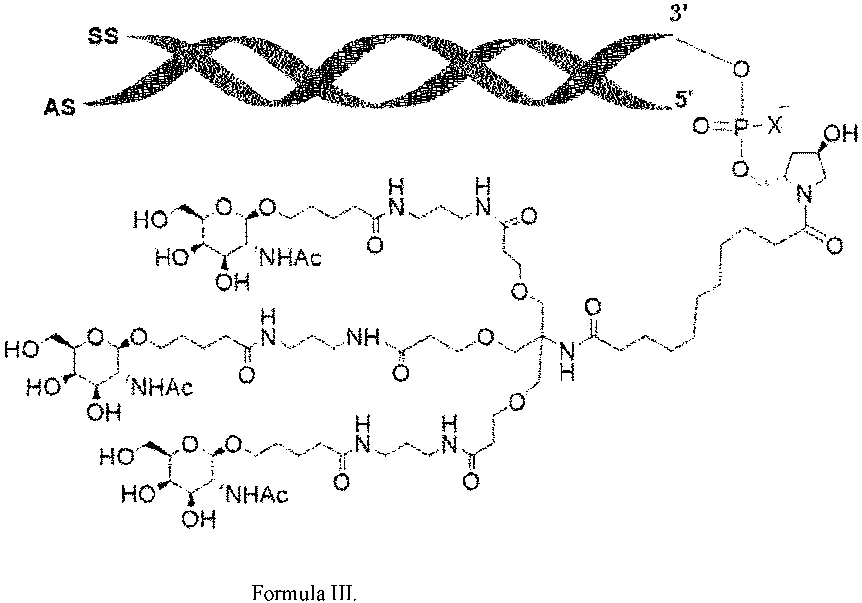

14. The double-stranded RNAi agent according to claim 11, wherein the double-stranded RNAi agent has:
(1) an antisense strand consisting of nucleotide sequence SEQ ID NO:3 and a sense strand consisting of nucleotide sequence SEQ ID NO:4;
(2) an antisense strand consisting of nucleotide sequence SEQ ID NO:5 and a sense strand consisting of nucleotide sequence SEQ ID NO:6;
(3) an antisense strand consisting of nucleotide sequence SEQ ID NO:7 and a sense strand consisting of nucleotide sequence SEQ ID NO:8;
(4) an antisense strand consisting of nucleotide sequence SEQ ID NO:9 and a sense strand consisting of nucleotide sequence SEQ ID NO:10;
(5) an antisense strand consisting of nucleotide sequence SEQ ID NO: 11 and a sense strand consisting of nucleotide sequence SEQ ID NO: 12;
(6) an antisense strand consisting of nucleotide sequence SEQ ID NO: 13 and a sense strand consisting of nucleotide sequence SEQ ID NO: 14;
(7) an antisense strand consisting of nucleotide sequence SEQ ID NO: 15 and a sense strand consisting of nucleotide sequence SEQ ID NO: 16;
(8) an antisense strand consisting of nucleotide sequence SEQ ID NO: 17 and a sense strand consisting of nucleotide sequence SEQ ID NO: 18;
(9) an antisense strand consisting of nucleotide sequence SEQ ID NO: 19 and a sense strand consisting of nucleotide sequence SEQ ID NO: 20;
(10) an antisense strand consisting of nucleotide sequence SEQ ID NO:21 and a sense strand consisting of nucleotide sequence SEQ ID NO:22;
(11) an antisense strand consisting of nucleotide sequence SEQ ID NO: 23 and a sense strand consisting of nucleotide sequence SEQ ID NO: 24;
(12) an antisense strand consisting of nucleotide sequence SEQ ID NO:25 and a sense strand consisting of nucleotide sequence SEQ ID NO:26;
(13) an antisense strand consisting of nucleotide sequence SEQ ID NO:27 and a sense strand consisting of nucleotide sequence SEQ ID NO:28;
(14) an antisense strand consisting of nucleotide sequence SEQ ID NO:29 and a sense strand consisting of nucleotide sequence SEQ ID NO:30;
(15) an antisense strand consisting of nucleotide sequence SEQ ID NO: 31 and a sense strand consisting of nucleotide sequence SEQ ID NO: 32;
(16) an antisense strand consisting of nucleotide sequence SEQ ID NO:33 and a sense strand consisting of nucleotide sequence SEQ ID NO:34;
(17) an antisense strand consisting of nucleotide sequence SEQ ID NO:35 and a sense strand consisting of nucleotide sequence SEQ ID NO:8;
(18) An antisense strand consisting of nucleotide sequence SEQ ID NO:36 and a sense strand consisting of nucleotide sequence SEQ ID NO:8;
(19) An antisense strand consisting of nucleotide sequence SEQ ID NO:37 and a sense strand consisting of nucleotide sequence SEQ ID NO:8;
(20) an antisense strand consisting of nucleotide sequence SEQ ID NO:38 and a sense strand consisting of nucleotide sequence SEQ ID NO:8;
(21) an antisense strand consisting of nucleotide sequence SEQ ID NO:39 and a sense strand consisting of nucleotide sequence SEQ ID NO:8;
(22) an antisense strand consisting of nucleotide sequence SEQ ID NO:40 and a sense strand consisting of nucleotide sequence SEQ ID NO:8;
(23) an antisense strand consisting of nucleotide sequence SEQ ID NO:7 and a sense strand consisting of nucleotide sequence SEQ ID NO:41;
(24) an antisense strand consisting of nucleotide sequence SEQ ID NO:42 and a sense strand consisting of nucleotide sequence SEQ ID NO:43;
(25) an antisense strand consisting of nucleotide sequence SEQ ID NO:44 and a sense strand consisting of nucleotide sequence SEQ ID NO:43;
(26) an antisense strand consisting of nucleotide sequence SEQ ID NO:44 and a sense strand consisting of nucleotide sequence SEQ ID NO:45;
(27) an antisense strand consisting of nucleotide sequence SEQ ID NO:46 and a sense strand consisting of nucleotide sequence SEQ ID NO:45;
(28) an antisense strand consisting of nucleotide sequence SEQ ID NO: 13 and a sense strand consisting of nucleotide sequence SEQ ID NO: 47;
(29) an antisense strand consisting of nucleotide sequence SEQ ID NO: 48 and a sense strand consisting of nucleotide sequence SEQ ID NO: 49;
(30) an antisense strand consisting of nucleotide sequence SEQ ID NO:50 and a sense strand consisting of nucleotide sequence SEQ ID NO:49;
(31) an antisense strand consisting of nucleotide sequence SEQ ID NO:50 and a sense strand consisting of nucleotide sequence SEQ ID NO:51;
(32) an antisense strand consisting of nucleotide sequence SEQ ID NO: 52 and a sense strand consisting of nucleotide sequence SEQ ID NO:51;
(33) an antisense strand consisting of nucleotide sequence SEQ ID NO: 53 and a sense strand consisting of nucleotide sequence SEQ ID NO:54;
(34) an antisense strand consisting of nucleotide sequence SEQ ID NO:53 and a sense strand consisting of nucleotide sequence SEQ ID NO:55; or
(35) an antisense strand consisting of nucleotide sequence SEQ ID NO: 53 and a sense strand consisting of nucleotide sequence SEQ ID NO: 56;
wherein Am, Um, Cm and Gm represent ribonucleotides A, U, C and G modified by 2'-O-methyl respectively; Af, Uf, Cf and Gf represent ribonucleotides A, U, C and G modified by 2'-fluoro respectively; s means that the two adjacent nucleotides are linked by a thiophosphate backbone., Cy5 means Cyanine 5 fluorescent dye, and L96 has the structure of formula IV: 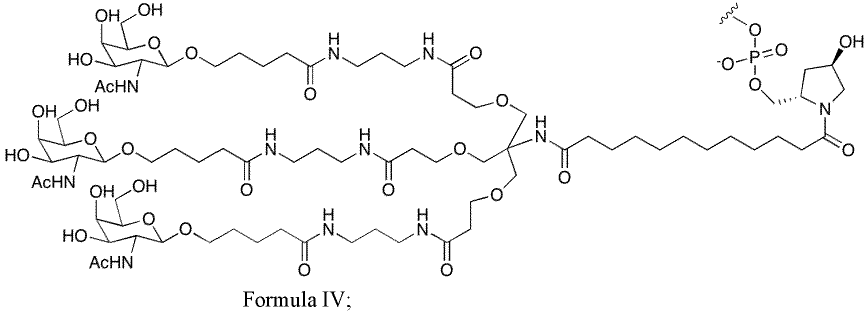
X has the structure of formula I: 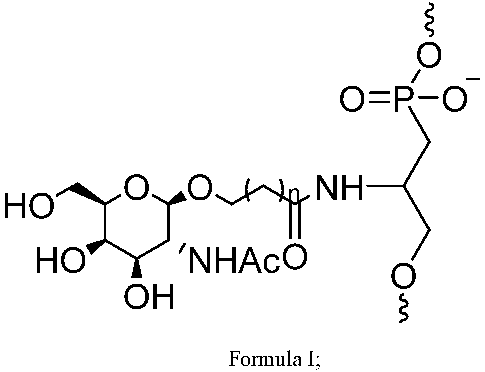
wherein n is 3.

15. A cell comprising the double-stranded RNAi agent according to any one of claims 1-14.

16. A pharmaceutical composition comprising the double-stranded RNAi agent according to any one of claims 1-15.

17. A method for inhibiting the expression of PCSK9 in a cell, which comprises: (a) contacting the cell with the double-stranded RNAi agent according to any one of claims 1-16 or the pharmaceutical composition according to claim 16; (b) maintaining the cell produced in step (a) for a period of time that is sufficient to obtain degradation of the mRNA transcript of the PCSK9 gene, thereby inhibiting the expression of the PCSK9 gene in the cell.

18. Use of the double-stranded RNAi agent according to any one of claims 1-14 or the pharmaceutical composition according to claim 16 in the manufacture of a medicament for treating a disease mediated by PCSK9 expression.

19. The use according to claim 18, wherein the disease comprises a cardiovascular disease or a neoplastic disease, and the cardiovascular disease is selected from the group consisting of hyperlipidemia, hypercholesterolemia, non-familial hypercholesterolemia, polygenic hypercholesterolemia, familial hypercholesterolemia, homozygous familial hypercholesterolemia or heterozygous familial hypercholesterolemia; the neoplastic disease is selected from PCSK9-related melanoma or metastatic liver cancer.
